(19) Europäisches Patentamt
European Patent Office
Office européen des brevets

(11) Publication number: **0 031 104**
**B1**

(12) # EUROPEAN PATENT SPECIFICATION

(45) Date of publication of patent specification: **23.10.85**

(51) Int. Cl.⁴: **C 07 D 277/06,** C 07 D 207/16, A 61 K 31/425, A 61 K 31/40

(21) Application number: **80107869.2**

(22) Date of filing: **12.12.80**

(54) **Thiazolidine compounds, processes for their preparation and pharmaceutical compositions containing them.**

(30) Priority: **13.12.79 JP 161977/79**

(43) Date of publication of application: **01.07.81 Bulletin 81/26**

(45) Publication of the grant of the patent: **23.10.85 Bulletin 85/43**

(84) Designated Contracting States: **AT BE CH DE FR GB IT LI NL SE**

(56) References cited:
EP-A-0 007 477
EP-A-0 012 401
EP-A-0 048 763
FR-A-2 023 741
FR-A-2 340 932
FR-A-2 340 933
FR-A-2 407 204
FR-A-2 412 537

**The file contains technical information submitted after the application was filed and not included in this specification**

(73) Proprietor: **SANTEN PHARMACEUTICAL CO., LTD.**
**9-19, 3-Chome, Shimoshinjo**
**Higashiyodogawa-ku Osaka 533 (JP)**

(72) Inventor: **Oya, Masayuki**
**27-18, 3-chome Yamatedai**
**Ibaraki-shi Osaka (JP)**
Inventor: **Iso, Tadashi**
**197-7, Joroku,**
**Sakai-shi Osaka (JP)**

(74) Representative: **Zumstein, Fritz jun., Dr. et al**
**Dr. F. Zumstein sen. Dr. E. Assmann Dr. R. Koenigsberger Dipl.-Ing. F. Klingseisen Dr. F. Zumstein jun. Bräuhausstrasse 4**
**D-8000 München 2 (DE)**

(56) References cited:
FR-A-2 434 150
FR-A-2 440 365
FR-A-2 445 324
GB-A-2 000 508
US-A-4 154 937

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 031 104 B1

**Description**

GB—A—2 000 508, FR—A—2 440 365 and FR—A—2 434 150 disclose inter alia special thiazolidine derivatives with an antihypertensive effect. FR—A—2 445 324 discloses inter alia special thiazolidine derivatives with an antihypertensive effect as a main effect goether with the effect to prevent or relieve diabetic complications as secondary effect. EP—A—12 401 discloses carboxyalkyl dipeptide derivatives which are useful only as antihypertensives.

This invention relates to thiazolidine compounds of the general formula

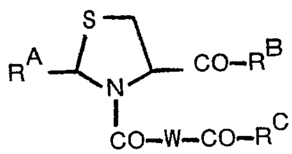

(I)

wherein

$R^A$ is $R^a$ or $R^b$;

$R^B$ and $R^C$ each is $R^c$;

W is

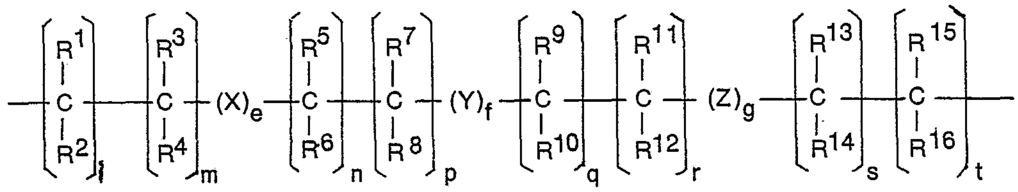

wherein

X, Y and Z are each

$-CH_2-$, 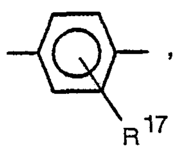 , 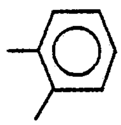 ,

$-O-$, $-CO-$, $-S-$, $-SO-$, $-SO_2-$, 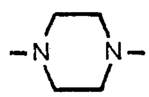 or 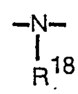 ;

whereby, when any of X, Y and Z is

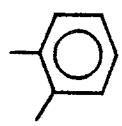 $R^A$ is 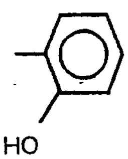 ,

$R^B$ and $R^C$ both are hydroxy and W is represented by

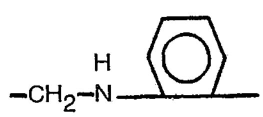 ;

e, f and g each is 0 or 1;

l, m, n, p, q, r, s and t are each 0, 1, 2 or 3, but (l+m) is not 0;

$R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$ and $R^{18}$ are each $R^d$;

when W is 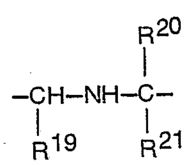 or 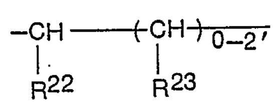

wherein $R^{19}$, $R^{20}$, $R^{21}$, $R^{22}$ and $R^{23}$ are each $R^d$, $R^A$ is $R^b$ when X is S and e is 1, (n+p) is not 0; when X is S, e is 1 and

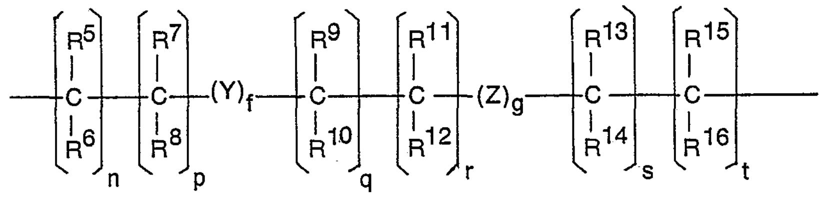

is lower alkylene $R^C$ is not hydroxy;

$R^a$ is hydrogen or $(C_1—C_4)$alkyl;

$R^b$ is selected from

(a) (i) benzyl or phenylethyl and

(ii) benzyl or phenylethyl substituted by at least one substituent selected from methyl, hydroxy, methoxy, halogen, nitro and cyano, and

(b) phenyl and phenyl substituted by at least one substituent selected from methyl, hydroxy, methoxy, benzyloxy, acetyloxy, halogen, nitro, cyano, amino, dimethylamino, carboxy, sulfamoyl, difluoromethoxy and dipropylaminosulfonyl;

$R^c$ is selected from

(a) hydroxy, $(C_1—C_4)$ alkoxy, amino, hydroxyamino and benzyloxy, and

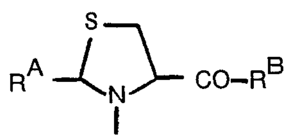
;
(b)

$R^d$ is selected from

(a) hydrogen, $(C_1—C_6)$ alkyl, benzyl, phenylethyl, acetyl, hydroxy, carboxy and amino, and

(b) phenyl and phenyl substituted by at least one substituent selected from methyl, methoxy, hydroxy, carboxy, amino, halogen, nitro and cyano, and salts thereof.

The thiazolidine compounds of the formula (I) are useful as agents for therapy or prophylaxis of the diabetic complication because they inhibit strongly aldose reductase.

Examples for the compounds of formula (I) or preferred groups of these compounds, respectively, are: Compounds of formula (I) wherein $R^a$ is hydrogen, methyl, ethyl, 1-methylethyl, propyl, 2-methylpropyl, butyl; compounds of formula (I) wherein $R^b$ is benzyl, 2-phenylethyl, 4-methylbenzyl, 4-methoxybenzyl, 2-hydroxybenzyl, 4-hydroxybenzyl, 3-fluorobenzyl, 3-nitrobenzyl, 3-cyanobenzyl, 2-(4-methoxyphenyl)ethyl, 2-(2-hydroxyphenyl)ethyl, 2-(4-hydroxyphenyl)ethyl, 2-(3-fluorophenyl)ethyl, 2-(3-nitrophenyl)ethyl, 2-(3-cyanophenyl)-ethyl, phenyl, 4-methylphenyl, 2-chlorophenyl, 4-chlorophenyl, 2,4-dichlorophenyl, 2-fluorophenyl, 3-fluorophenyl, 4-fluorophenyl, 2-nitrophenyl, 3-nitrophenyl, 4-nitrophenyl, 2-chloro-5-nitrophenyl, 4-dimethyl-aminophenyl, 2-carboxyphenyl, 4-carboxyphenyl, 2-hydroxyphenyl, 3-hydroxyphenyl, 4-hydroxyphenyl, 3-benzoxyphenyl, 3,4-dihydroxyphenyl, 5-chloro-2-hydroxyphenyl, 2-methoxyphenyl, 4-methoxyphenyl, 3,4-dimethoxyphenyl, 3,4,5-trimethoxyphenyl 2-hydroxy-3-methoxyphenyl, 2-hydroxy-4-methoxyphenyl, 4-hydroxy-3-methoxyphenyl, 2-cyanophenyl, 3-cyanophenyl, 4-cyanophenyl, 2-hydroxy-5-sulfamoylphenyl, 2-hydroxy-5-[(dipropylamino)sulfonyl]phenyl, 3-(difluoromethoxy)phenyl; compounds of formula (I) wherein $R^c$ is hydroxy, methoxy, ethoxy, butoxy, amino, hydroxyamino, benzyloxy or

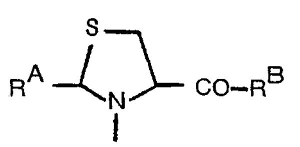
;

compounds of formula (I) wherein $R^d$ is hydrogen, methyl, ethyl, propyl, 1-methylethyl, 2-methylpropyl, 4-methylpentyl, cyclohexyl, benzyl, 2-phenylethyl, phenyl, 4-methyphenyl, 2-chlorophenyl, 4-chlorophenyl, 2,4-dichlorophenyl, 2-fluorophenyl, 3-fluorophenyl, 4-fluorophenyl, 2-nitrophenyl, 3-nitrophenyl, 4-nitrophenyl, 2-chloro-5-nitrophenyl, 2-carboxyphenyl, 4-carboxyphenyl, 2-hydroxyphenyl, 3-hydroxyphenyl, 4-hydroxyphenyl, 3,4-dihydroxyphenyl, 5-chloro-2-hydroxyphenyl, 2-methoxyphenyl, 4-methoxyphenyl, 3,4-dimethoxyphenyl, 3,4,5-trimethoxyphenyl, 2-hydroxy-3-methoxyphenyl; compounds of formula (I) wherein W is

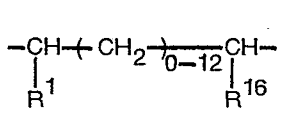,
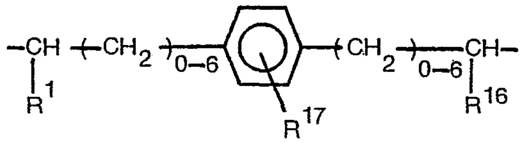,
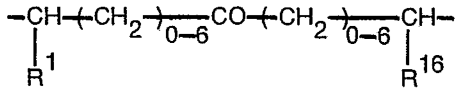,
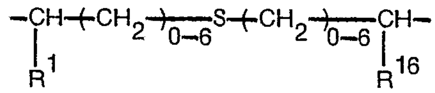,
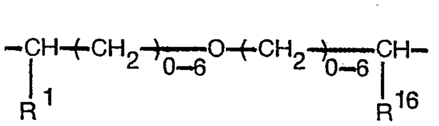,
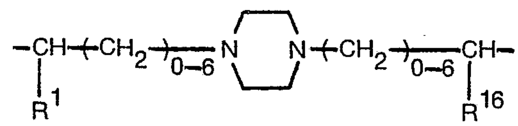,
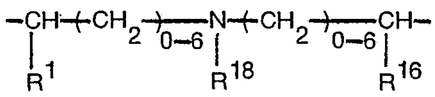,
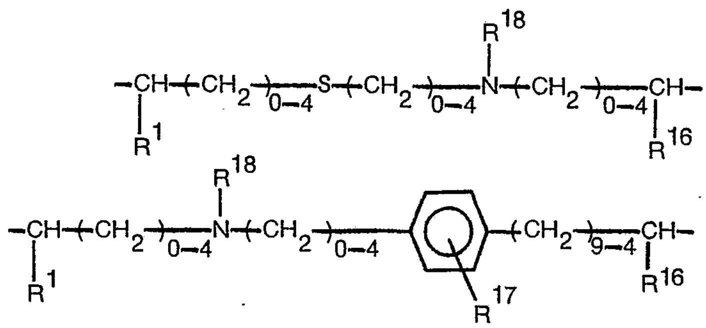

or 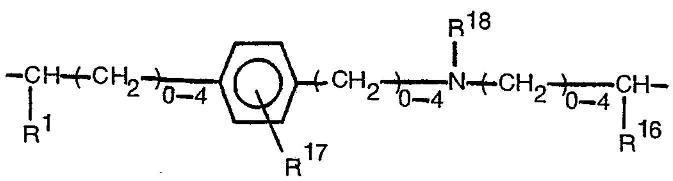.

Additionally to the compounds described in the examples the following compound is comprised by the compounds of the invention: (4R)-3-[[[1-(ethoxycarbonyl)-3-phenylpropyl]amino]acetyl]-2-(2-hydroxyphenyl)-4-thiazolidine-carboxylic acid.

The compounds [I] of this invention can be prepared by following process.

(i) A compound of the formula (I) is yielded by the reaction of a compound of the formula [II]

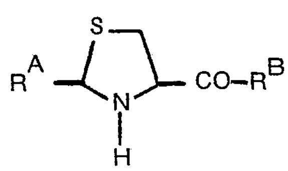

(II)

wherein $R^A$ and $R^B$ may be protected by any suitable groups (e.g., lower alkyl, acyl, aralkyl, aralkyloxy) when $R^A$ and $R^B$ include reactive groups (e.g., amino, hydroxy, hydroxyamino), with the reactive derivative of carboxylic acid of the formula [III] (e.g., acyl halide, acid anhydride, mixed anhydride, active ester, lactone) by general methods used in peptide syntheses or amide formation reactions

$$HOOC—W—COR^C \qquad [III]$$

wherein W and $R^C$ may be protected by any suitable groups (e.g., lower alkyl, acyl, aralkyl, aralkyloxy) when W and $R^C$ include reactive groups (e.g., amino, hydroxy, hydroxyamino), followed by removal of protective groups by well-known methods (e.g., hydrolysis, hydrogenolysis, ammonolysis, alcoholysis).

This procedures of deprotection can be applied in the following methods.

(ii) A compound of the formula [I] is yielded by the reaction of a compound of the formula [II] with the reactive derivative of carboxylic acid of [IV] (e.g., above-mentioned)

$$HOOC—W^1—L \qquad [IV]$$

wherein

$W^1$ is 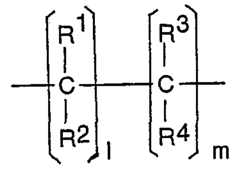 ,

and may be protected such as (i) above, L is a leaving group (e.g., halogen, alkylsulfonyl, arylsulfonyl), by the same methods as described in

(i) above to produce a compound of the formula [V]

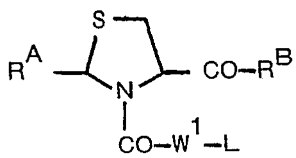 (V)

and then reaction of a compound of the formula [V] with a compound of the formula [VI]

$$(H)(X)_e W^2—(Y)_f—W^3—(Z)_g—W^4—CO—R^C \quad [VI]$$

wherein

$W^2$ is 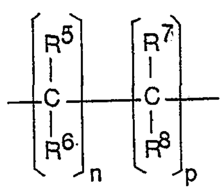 , $W^3$ is 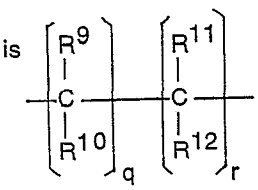 , $W^4$ is 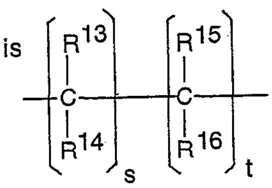

and $W^2$, $W^3$, $W^4$, X, Y, Z and $R^C$ may be protected such as (i) above, in the presence of proper alkaline and/or organic bases, if necessary, by known methods.

(iii) A compound of the formula [I] is yielded by the reaction of a compound of the formula [II] with the reactive derivative of carboxylic acid of the formula [VII] (e.g., mentioned in (i) above)

$$HOOC—W^1—(X)_e—W^2—L \quad [VII]$$

and then with a compound of the formula (VIII)

$$(H)(Y)_f—W^3—(Z)_g—W^4—CO—R^C \quad [VIII]$$

by the same methods as (ii) above.

(iv) A compound of the formula [I] is yielded by the reaction of a compound of the formula [II] with the reactive derivative of carboxylic acid of the formula [IX] (e.g., mentioned in (i) above)

$$HOOC—W^1—(X)_e—W^2—(Y)_f—W^3—L \quad [IX]$$

and then with a compound of the formula [X]

$$(H)(Z)_g—W^4—CO—R^C \quad [X]$$

by the same method as (ii) above.

(v) A compound of the formula [I] is yielded by the reaction of a compound of the formula [II] with the reactive derivative of carboxylic acid [XI] (e.g., acyl halide, acid anhydride, mixed anhydride, active ester, lactone, thiolactone)

$$HOOC—W^1—[(X)_e(H) \quad [XI]$$

and then with a compound of the formula [XII]

$$L—W^2—(Y)_f—W^3—(Z)_g—W^4—CO—R^C \qquad [XII]$$

by the same method as (ii) above.

(vi) A compound of the formula [I] is yielded by the reaction of a compound of the formula [II] with the reactive derivative of carboxylic acid of the formula [XIII] (e.g., mentioned in (v) above)

$$HOOC—W^1—(X)_e—W^2—(Y)_f(H) \qquad [XIII]$$

and then with a compound of the formula [XIV]

$$L—W^3—(Z)_g—W^4—CO—R^C \qquad [XIV]$$

by the same method as (ii) above.

(vii) A compound of the formula [I] is yielded by the reaction of a compound of the formula [II] with the reactive derivative of carboxylic acid of the formula [XV] (e.g., mentioned in (v) above)

$$HOOC—W^1—(X)_e—W^2—(Y)_f—W^3—(Z)_g(H) \qquad [XV]$$

and then with a compound of the formula [XVI]

$$L—W^4—CO—R^C \qquad [XVI]$$

by the same method as (ii) above.

(viii) A compound of the formula [I] is also yielded by converting a compound of the formula [I] prepared by any method above-mentioned by well-known methods (e.g., oxidation, formation of oxime, hydrazone and semicarbazone, addition to double bond).

The compounds [I] of this invention are effective on preventing or relieving diabetic complications.

In diabetic patients, high levels of hexoses (e.g., glucose, galactose) in blood lead to the accumulation of sugar alcohols (e.g., sorbitol, galactitol) in tissues. It is known that this accumulation causes the swelling of cells to induce complications of diabetic cataract, diabetic retinopathy, diabetic nephropathy, diabetic neuropathy, etc. [R. Quan-Ma et al., Biochem. Biophys. Res. Comm., *22,* 492 (1966)]. For example, R. Gitzelman et al. have presented that cataract is caused by the accumulation of sugar alcohols [Exptl. Eye. Res., *6,* 1 (1967)]. A report of Kinoshita et al, has demonstrated that aldose reductase which reduced aldose to the corresponding sugar alcohols was involved in the initiation of these diabetic complications and that effective inhibitors of aldose reductase were useful [Jpn. J. Ophthalmol., *20,* 339 (1976)]. On the basis of the above information, aldose reductase inhibition of the compounds [I] of this invention was tested. The results of the examinations demonstrated that these compounds have potent inhibitory activities on aldose reductase, and therefore they are useful as drugs for therapy or prophylaxis of the diabetic complications.

The compound of formula (I) can form the conventional salts to be generally used as medicine such as sodium salt, potassium salt, calcium salt, magnesium salt, aluminum salt, ammonium salt, diethylamine salt and triethanolamine.

The compounds of formula [I] have the stereoisomers which are within the limit of this invention, because they have one or more asymmetric carbon atoms.

Typical examples are shown below.

### Example 1

(4R)-3-(7-Carboxyheptanoyl)-2-(2-hydroxyphenyl-4-thiazolidinecarboxylic acid (compound 20)

(4R)-2-(2-Hydroxyphenyl)-4-thiazolidinecarboxylic acid (6.8 g,) in N sodium hydroxide (30 ml) and octanedioyl dichloride (6.3 g,) were added dropwise to 1M potassium carbonate (60 ml) with stirring under ice-cooling. After the addition, the reaction mixture was stirred for 1 hour at the same temperature and for additional 1 hour at room temperature. The solution was acidified with dilute hydrochloric acid, and extracted with ethyl acetate. The organic layer was washed with saturated sodium chloride solution, dried over anhydrous magnesium sulfate, and evaporated in vacuo. The residual oil*2 was purified by silica gel column chromatography to give 7.0 g (61%) of the titled compound: mp 155—157°C (dec.) (ethyl acetate); $[\alpha]_D^{27}$ +134.1° (c=0.5, methanol). IR (nujol, $cm^{-1}$): 3220 (OH), 1710 (COOH), 1620 (CON), 1600 (aromatic), 1415, 1235, 1172, 950, 760, NMR (DMSO-$d_6$, δ): 0.53—1.73 (8H, m, $—CH_2—(CH_2)_4—CH_2—$), 1.77—2.57 (4H, m, $—\underline{CH_2}—(CH_2)_4—\underline{CH_2}—$), 3.03 (1H, $AB_q$ (A part), d, J=11.5, 8.5Hz, C*.511[1]—$H_A$), 3.37 (1H, $AB_q$ (B part), d, J=11.5, 6.5Hz, $C_5—H_B$), 4.60 (1H, dd, J=8.5, 6.5Hz, $C_4$—H), 6.28 (1H, s, $C_2$—H), 6.45—8.07 (4H, m, arom. H), 9.77 (1H, s, —COO<u>H</u>). TLC: Rf value*3 0.52.

---

*1 The numbers represent the positions on thiazolidine or pyrrolidine ring. The same shall be applied hereinafter..

*2 Two spots were observed on the TLC (ethyl acetate-chloroform-acetic acid (10:5:3)), and two products could be separated by silica gel column chromatography. From NMR spectra, the upper and lower spots were identified as the titled compound and (4R,4R')-3,3'-(octanedioyl)bis[2-(2-hydroxyphenyl)-4-thiazolidinecarboxylic acid] (compound 40), respectively.

*3 Silica gel, ethyl acetate-chloroform-acetic acid (10:5:3).

The compounds shown in Table I and III were prepared by the same procedure as described above. The following compounds are also prepared by the same procedure as EXAMPLE 1.

(4R)-3-carboxyacetyl-4-thiazolidinecarboxylic acid
(4R)-3-(3-carboxypropanoyl)-2-phenyl-4-thiazolidinecarboxylic acid
(4R)-3-[3-(2-carboxyethylsulfinyl)propanoyl]-2-(2-hydroxyphenyl)-4-thiazolidinecarboxylic acid
(4R)-3-[[[2-(carboxymethyloxy)ethyl]oxy]acetyl]-2-(2-hydroxyphenyl)-4-thiazolidinecarboxylic acid
(4R)-3-(4-carboxybutanoyl)-2-(3-hydroxyphenyl)-4-thiazolidinecarboxylic acid
(4R)-3-(5-carboxypentanoyl)-2-(4-methylphenyl)-4-thiazolidinecarboxylic acid
(4R)-3-(6-carboxyhexanoyl)-2-(4-chlorophenyl)-4-thiazolidinecarboxylic acid
(4R)-3-(7-carboxyheptanoyl)-2-(4-methoxyphenyl)-4-thiazolidinecarboxylic acid
(4R)-3-(13-carboxytridecanoyl)-2-(2-nitrophenyl)-4-thiazolidinecarboxylic acid
(4R)-3-(7-carboxyheptanoyl)-2-(3-nitrophenyl)-4-thiazolidinecarboxylic acid
(4R)-3-[3-(2-carboxyethylthio)propanoyl]-2-(3-nitrophenyl)-4-thiazolidinecarboxylic acid
(4R)-3-[[[2-(carboxymethyloxy)ethyl]oxy]acetyl]-2-(3-nitrophenyl)-4-thiazolidinecarboxylic acid
(4R)-3-(6-carboxyhexanoyl)-2-(4-nitrophenyl)-4-thiazolidinecarboxylic acid
(4R)-3-(9-carboxynonanoyl)-2-(4-nitrophenyl)-4-thiazolidinecarboxylic acid
(4R)-3-(11-carboxyundecanoyl)-2-(4-nitrophenyl)-4-thiazolidinecarboxylic acid
(4R)-3-[4-(3-carboxypropyloxy)butanoyl]-2-(4-nitrophenyl)-4-thiazolidinecarboxylic acid
(4R)-3-[3-(2-carboxyethylsulfonyl)propanoyl]-2-[4-nitrophenyl)-4-thiazolidinecarboxylic acid
(4R)-3-(9-carboxynonanoyl)-2-(5-chloro-2-hydroxyphenyl)-4-thiazolidinecarboxylic acid
(4R)-3-(11-carboxyundecanoyl)-2-(3,4,5-trimethoxyphenyl)-4-thiazolidinecarboxylic acid
(4R)-3-(13-carboxytridecanoyl)-2-(2-acetoxyphenyl)-4-thiazolidinecarboxylic acid
(4R)-3-(6-carboxyhexanoyl)-2-(2-furyl)-4-thiazolidinecarboxylic acid
(4R)-3-(7-carboxyheptanoyl)-2-(2-thienyl)-4-thiazolidinecarboxylic acid
(4R)-3-(8-carboxyoctanoyl)-2-(3-pyridyl)-4-thiazolidinecarboxylic acid
(4R)-3-(9-carboxynonanoyl)-2-(1-naphthyl)-4-thiazolidinecarboxylic acid
(4R)-3-(5-carboxypentanoyl)-2-(2-hydroxy-4-sulfamoylphenyl)-4-thiazolidinecarboxylic acid
(4R)-3-(6-carboxyhexanoyl)-2-(3-cyanophenyl)-4-thiazolidinecarboxylic acid
(4R)-3-(7-carboxyheptanoyl)-2-(3-difluoromethoxyphenyl)-4-thiazolidinecarboxylic acid
(4R)-3-(8-carboxyoctanoyl)-2-(4-carboxyphenyl)-4-thiazolidinecarboxylic acid
(4R)-3-(9-carboxynonanoyl)-2-(3-methylsulfinylphenyl)-4-thiazolidinecarboxylic acid

## Example 2

(4R,4′R)-3,3′-(Octanedioyl)bis[2-(2-hydroxyphenyl)-4-thiazolidinecarboxylic acid (compound 40)

To a stirred solution of (4R)-2-(2-hydroxyphenyl)-4-thiazolidinecarboxylic acid (6.8 g) in 1M potassium carbonate (45 ml), octanedioyl dichloride (3.2 g) was added dropwise under ice-cooling. After the addition, the reaction mixture was stirred for 1 hour at the same temperature and for additional 1 hour at room temperature. The solution was acidified with dilute hydrochloric acid, extracted with ethyl acetate. The organic layer was washed with saturated sodium chloride solution, dried over anhydrous magnesium sulfate, and evaporated in vacuo. The residual oil was purified by silica gel column chromatography to give 7.6 g (86%) of the titled compound: mp 93—97°C (dec.); $[\alpha]_D^{27}$ +123.6° (c=0.5, methanol). IR (nujol. $cm^{-1}$): 1720 (COOH), 1620 (CON), 1600 (aromatic), 1230, 1090, 855, 765. MNR ($CD_3OD$) δ: 0.7—1.7 (8H, m, $-CH_2-(CH_2)_4-CH_2-$), 1.8—2.4 (4H, m, $-CH_2-(CH_2)_4-CH_2$), 3.25 (4H, d, J=7.5Hz, $C_5$—H), 4.81 (2H, t, J=7.5Hz, $C_4$—H), 6.35 (2H, s, $C_2$—H), 6.7—8.0 (8H, m, arom. H). TLC: Rf value* 0.34.

The compounds shown in Table II and III were prepared by the same procedure as described above.

## Example 3

(4R,4′R)-3,3′-(heptanedioyl)bis[2-(3-cyanophenyl)-4-thiazolidinecarboxylic acid] (compound 36)

To a stirred solution of (4R)-2-(3-cyanophenyl)-4-thiazolidinecarboxylic acid (4.7 g) in 1M sodium carbonate (30 ml), heptanedioyl dichloride (2.1 g) was added dropwise under ice-cooling. The reaction mixture was stirred for 30 minutes at the same temperature, and filtered to give the precipitates. The precipitates were dissolved in hot water (100 ml), and acidified with concentrated hydrochloric acid. The separated crystals were collected by filtration to give 3.5 g (59%) of the titled compound: mp 105—112°C; $[\alpha]_D^{25}$ +115.0° (c=1.0, methanol). IR (nujol, $cm^{-1}$): 2270 (CN), 1735 (COOH), 1640 (CON), 1610 (aromatic), 1195, 790 (aromatic). NMR (DMSO-$d_6$) δ: 0.69—1.66 (6H, m, $-CH_2-(CH_2)_3-CH_2-$), 1.70—2.50 (4H, m, $-CH_2-(CH_2)_3-CH_2-$), 2.85—3.66 (4H, m, $C_5$—H), 4.69 (1H, dd, J=8.2, 6.0Hz, $C_4$—H), 5.13 (1H, m, $C_4$—H), 6.16 (1H, s, $C_2$—H), 6.43 (1H, s, $C_2$—H), 7.3—8.3 (8H, m, arom. H). TLC: Rf value* 0.33.

* Silica gel, ethyl acetate-chloroform-acetic acid (10:5:3)

The compounds shown in Table II were prepared by the same procedure as described above.
The following compounds are also prepared by the same procedure as EXAMPLE 2 or 3.
(4R,4′R)-3,3′-(propanedioyl)bis(4-thiazolidinecarboxylic acid)
(4R,4′R)-3,3′-(butanedioyl)bis(2-phenyl)-4-thiazolidinecarboxylic acid)
(4R,4′R)-3,3′-(3,3′-sulfinyldipropanoyl)bis[2-(2-hydroxyphenyl)-4-thiazolidinecarboxylic acid]
(4R,4′R)-3,3′-[(ethylenedioxy)diacetyl]bis[2-2-hydroxyphenyl)-4-thiazolidinecarboxylic acid
(4R,4′R)-3,3′-[(ethylenedithio)diacetyl]bis[2-(2-hydroxyphenyl)-4-thiazolidinecarboxylic acid
(4R,4′R)-3,3′-(pentanedioyl)bis[2-(3-hydroxyphenyl)-4-thiazolidinecarboxylic acid]
(4R,4′R)-3,3′-(hexanedioyl)bis[2-(4-methylphenyl)-4-thiazolidinecarboxylic acid]
(4R,4′R)-3,3′-(heptanedioyl)bis[2-(4-chlorophenyl)-4-thiazolidinecarboxylic acid]
(4R,4′R)-3,3′-(octanedioyl)bis[2-(4-methoxyphenyl)-4-thiazolidinecarboxylic acid]
(4R,4′R)-3,3′-(tetradecanedioyl)bis[2-(2-nitrophenyl)-4-thiazolidinecarboxylic acid]
(4R,4′R)-3,3′-(3,3′-thiodipropanoyl)bis[2-(3-nitrophenyl)-4-thiazolidinecarboxylic acid]
(4R,4′R)-3,3′-[(ethylenedioxy)diacetyl]bis[2-(3-nitrophenyl)-4-thiazolidinecarboxylic acid]
(4R,4′R)-3,3′-(heptanedioyl)bis[2-(4-nitrophenyl)-4-thiazolidinecarboxylic acid]
(4R,4′R)-3,3′-(decanedioyl)bis[2-(4-nitrophenyl)-4-thiazolidinecarboxylic acid]
(4R,4′R)-3,3′-(dodecanedioyl)bis[2-(4-nitrophenyl)-4-thiazolidinecarboxylic acid]
(4R,4′R)-3,3′-(4,4′-oxydibutanoyl)bis[2-(4-nitrophenyl)-4-thiazolidinecarboxylic acid]
(4R,4′R)-3,3′-(3,3′-sulfonyldipropanoyl)bis[2-(4-nitrophenyl)-4-thiazolidinecarboxylic acid]
(4R,4′R)-3,3′-(decanedioyl)bis[2-(5-chloro-2-hydroxyphenyl)-4-thiazolidinecarboxylic acid]
(4R,4′R)-3,3′-(dodecanedioyl)bis[2-(3,4,5-trimethoxyphenyl)-4-thiazolidinecarboxylic acid]
(4R,4′R)-3,3′-(tetradecanedioyl)bis[2-(2-acetoxyphenyl)-4-thiazolidinecarboxylic acid]
(4R,4′R)-3,3′-(heptanedioyl)bis[2-(2-furyl)-4-thiazolidinecarboxylic acid]
(4R,4′R)-3,3′-(octanedioyl)bis[2-(2-thienyl)-4-thiazolidinecarboxylic acid]
(4R,4′R)-3,3′-(nonanedioyl)bis[2-(3-pyridyl)-4-thiazolidinecarboxylic acid]
(4R,4′R)-3,3′-(decanedioyl)bis[2-(1-naphtyl)-4-thiazolidinecarboxylic acid]
(4R,4′R)-3,3′-(hexanedioyl)bis[2-(2-hydroxy-5-sulfamoylphenyl)-4-thiazolidinecarboxylic acid]
(4R,4′R)-3,3′-(octanedioyl)bis[2-(3-difluoromethoxyphenyl)-4-thiazolidinecarboxylic acid]
(4R,4′R)-3,3′-(nonanedioyl)bis[2-(4-carboxyphenyl)-4-thiazolidinecarboxylic acid]
(4R,4′R)-3,3′-(decanedioyl)bis[2-(3-methylsulfinylphenyl)-4-thiazolidinecarboxylic acid]

Example 4

(4R,4′R)-3,3′-(Heptanedioyl)bis[2-(3-nitrophenyl)-4-thiazolidinecarboxylic acid] (compound 35)

To a stirred solution of (4R)-2-(3-nitrophenyl)-4-thiazolidinecarboxylic acid (5.1 g) in 1M sodium carbonate (40 ml), heptanedioyl dichloride (2.1 g) was added dropwise under ice-cooling. The reaction mixture was stirred for 1 hour at the same temperature, and the separated crystals were filtered to give 4.7 g (69%) of the titled compound as disodium salt: mp 111—113°C (dec.) (water); $[\alpha]_D^{25}$ +88.2° (c=0.5, methanol). IR (nujol, $cm^{-1}$): 1635 (CON) 1585 ($COO^-$), 1520 and 1355 ($NO_2$), 1095, 730. TLC: Rf value* 0.28.

Example 5

(4R)-3-(3-Carboxypropanoyl)-2-(2-hydroxyphenyl)-4-thiazolidinecarboxylic acid (compound 6)

To a stirred solution of (4R)-2-(2-hydroxyphenyl)-4-thiazolidinecarboxylic acid (4.5 g) and triethylamine (4.0 g) in acetone (100 ml), succinic anhydride (2.0 g) was added at room temperature, and stirred for 3 hours at the same temperature. The reaction mixture was concentrated in vacuo, and acidified with dilute hydrochloric acid. The separated oil was extracted with ethyl acetate, and the organic layer was washed with saturated sodium chloride solution, dried over anhydrous magnesium sulfate, and evaporated in vacuo to give 4.9 g (75%) of the titled compound: mp 190—191°C (dec.) (ethyl acetate-methanol); $[\alpha]_D^{27}$ +181.6° (c=1.0, methanol). IR (nujol, $cm^{-1}$): 3210 (OH), 1720 (COOH), 1618 (CON), 1602 (aromatic), 1245, 1173, 940, 763. NMR (DMSO-$d_6$, δ): 2.0—2.7 (4H, m, —$CH_2CH_2$—), 3.03 (1H, $AB_q$ (A part), d, J=11.0, 10.0Hz, $C_5$—$H_A$), 3.36 (1H, $AB_q$ (B part), d, J=11.0, 7.0Hz, $C_5$—$H_B$), 4.61 and 5.07 (1H, dd, J=10.0, 7.0Hz and m, $C_4$—H), 6.36 (1H, s, $C_2$—H), 6.5—8.0 (4H, arom. H). TLC: Rf value* 0.35.

The compounds shown in Table I and III were prepared by the same procedure as described above. The following compounds are also prepared by the same procedure as EXAMPLE 5.
(4R)-3-(4-carboxy-4-oxobutanoyl)-2-(2-hydroxyphenyl)-4-thiazolidinecarboxylic acid
(4R)-3-(6-carboxy-3,5-dioxohexanoyl)-2-(2-hydroxyphenyl)-4-thiazolidinecarboxylic acid
(4R)-3-[4-carboxy-3-(methoxyimino)butanoyl]-2-(2-hydroxyphenyl)-4-thiazolidinecarboxylic acid.

Example 6

(4R)-3-[3-(Methoxycarbonyl)-2-methylpropanoyl]-2-(2-hydroxyphenyl)-4-thiazolidinecarboxylic acid (compound 4)

To a stirred solution of (4R)-2-(2-hydroxyphenyl)-4-thiazolidinecarboxylic acid (11.3 g) in 1M sodium carbonate (80 ml), dl-3-methoxycarbonyl-2-methylpropanoyl chloride (8.2 g) was added dropwise under ice-cooling.

---

* Silica gel, ethyl acetate-chloroform-acetic acid (10:5:3).

After the addition, the reaction mixture was stirred for 1.5 hours at the same temperature. After the filtration of solution, the filtrate was acidiffied with dilute hydrochloric acid, and extracted with ethyl acetate. The organic layer was washed with saturated sodium chloride solution, dried over anhydrous magnesium sulfate, and evaporated in vacuo. The residual oil was purified by silica gel column chromatography to give 7.8 g (44%) of the titled compound: $[\alpha]_D^{25}$ +161.6° (c=1.0, methanol). IR (KBr, $cm^{-1}$): 3380 (OH), 1723 (COOH, $COOCH_3$), 1624 (CON), 1235, 1200, 1174, 764.

The compounds shown in Table I and II were prepared by the same procedure as described above.

## Example 7

(4R)-3-(3-Carboxy-2-methylpropanoyl)-2-(2-hydroxyphenyl)-4-thiazolidinecarboxylic acid (compound 5)

(4R)-3-[3-(Methoxycarbonyl)-2-methylpropanoyl]-2-(2-hydroxyphenyl)-4-thiazolidinecarboxylic acid (compound 4) (7.1 g) was dissolved in 2N sodium hydroxide (40 ml) and stirred for 1 hour at room temperature. The resulting solution was acidified with dilute hydrochloric acid and the separated crystals were filtered to give 5.1 g (75%) of the titled compound: mp 163—164°C (dec.) (ethyl acetate); $[\alpha]_D^{25}$ +174.1° (c=1.0, methanol). IR (nujol, $cm^{-1}$): 3330 (OH), 1730 and 1710 (COOH), 1629 (CON), 1280, 1234, 856, 771.

The compounds shown in Table I and II were prepared by the same procedure as described above. The following compounds are also prepared by the same procedure as EXAMPLE 6 and 7.

(4R)-3-(4-(carboxymethyl)benzoyl]-2-(2-hydroxyphenyl)-4-thiazolidinecarboxylic acid
(4R)-3-[(4-carboxyphenyl)acetyl]-2-phenyl-4-thiazolidinecarboxylic acid
(4R)-3-(4-carboxy-3-butenoyl)-2-(2-hydroxyphenyl)-4-thiazolidinecarboxylic acid
(4R)-3-(4-carboxy-2-butenoyl)-2-(2-hydroxyphenyl)-4-thiazolidinecarboxylic acid
(4R)-3-(4-carboxy-3-butynoyl)-2-(2-hydroxyphenyl)-4-thiazolidinecarboxylic acid

## Example 8

(4R)-3-[3-(N-Hydroxycarbamoyl)propanoyl]-2-(2-hydroxyphenyl)-4-thiazolidinecarboxylic acid ethyl ester (compound 10a)

To a stirred solution of (4R)-3-(3-carboxypropanoyl)-2-(2-hydroxyphenyl)-4-thiazolidinecarboxylic acid ethyl ester (compound 8a) (1.06 g) and N-methylmorpholine (0.33 ml) in 20 ml of anhydrous tetrahydrofuran, isobutyl chloroformate (0.39 ml) was added dropwise at −15°C, and stirred for additional 2 hours at this temperature. To this solution, the methanol solution of hydroxylamine (0.3 g) was added dropwise at −50°C. The reaction mixture was stirred for 1 hour at room temperature, acidified with N hydrochloric acid, and extracted with ethyl acetate. The organic layer was washed with saturated sodium chloride solution, dried over anhydrous magnesium sulfate, and concentrated in vacuo. The residual oil was purified by silica gel column chromatography to give 0.7 g (63%) of the titled compound. IR (KBr, $cm^{-1}$) 3220, 1727, 1625, 1595, 1200, 1092, 753. NMR (acetone-$d_6$, δ): 1.24 (3H, t, J=7.5Hz, $CO_2CH_2\underline{CH_3}$), 2.17—3.07 (4H, m, $CO{-}(\underline{CH_2})_2{-}CO$), 3.30 (1H, $AB_q$ (A part), d, J=10.0, 2.0Hz, $C_5{-}H_A$), 3.47 (1H, $AB_q$ (B part), d, J=10.0, 7.0Hz, $C_5{-}H_B$), 4.14 (2H, q, J=7.5Hz, $CO_2\underline{CH_2}$), 5.18 (1H, dd, J=2.0, 7.0Hz, $C_4{-}H$), 6.40 (1H, S, $C_2{-}H$), 6.88—7.27 (4H, m, arom. H), 8.60 (2H, br. s, N$\underline{H}$O$\underline{H}$), 9.77 (1H, br. s, O$\underline{H}$)

The compounds shown in Table I were prepared by the same procedure as described above.

## Example 9

(4R,4′R)-3,3′-(Nonanedioyl)bis[2-(3-nitrophenyl)-4-thiazolidinecarboxylic acid methyl ester] (compound 46)

To a stirred solution of (4R,4′R) - 3,3′ - (nonanedioyl)bis - [2 - (3 - nitrophenyl) - 4 - thiazolidinecarboxylic acid] (compound 47) (3.3 g) in ethyl acetate (50 ml), 2% ether solution of diazomethane was added dropwise until the yellow color of diazomethane was not disappeared, and stirred continuously for 30 minutes. The reaction mixture was concentrated in vacuo to give 3.3 g (96%) of the titled compound: mp 61—63°C (benzene); $[\alpha]_D^{23}$ +79.4° (c=1.0, methanol). IR (KBr, $cm^{-1}$): 1740, 1660, 1530, 1350, 1198, 725.

## Example 10

(4R)-3-[(2-Carboxymethylthio-3-phenyl)propanoyl]-4-thiazolidinecarboxylic acid (compound 74a and 74b)

(4R)-3-[(2-Mercapto-3-phenyl)propanoyl]-4-thizolidinecarboxylic acid (1.0 g), potassium carbonate (0.7 g), chloroacetic acid (0.2 g) and potassium iodide (0.05 g) were dissolved in water (5 ml), and stirred for 6 hours at room temperature. The reaction mixture was acidified with 5N hydrochloric acid and extracted with ethyl acetate. The organic layer was washed with saturated sodium chloride solution, dried over anhydrous magnesium sulfate and concentrated in vacuo. The titled compounds (74a and 74b) were separated from the oily residue by silica gel column chromatography.

| | 75a | 75b |
|---|---|---|
| yield | 0.4g (37%) | 0.5g (47%) |
| $[\alpha]_D^{25}$ | −52.2° (c=1.2, MeOH) | −60.4° (c=1.0, MeOH) |
| IR<br>(neat, $cm^{-1}$) | 1720, 1620, 1422,<br>1217, 756 | 1722, 1620, 1420,<br>1215, 755 |
| NMR<br>($CDCl_3$, δ) | 2.67−3.63 (6H, m,<br>$-S-C\underline{H_2}-CO_2H$, $C_5-H$,<br>$-C\underline{H_2}-Ph$),<br>3.83−4.83 (3H, m,<br>$-CO-C\underline{H}-S-$, $C_2-H$),<br>4.98 (1H, dd, J=4.5,<br>6.5Hz, $C_4-H$),<br>7.22 (5H, s, $-C_6H_5$)<br>9.55 ($-CO_2H$) | 2.70−3.50 (6H, m,<br>$-S-C\underline{H_2}-CO_2H$, $C_5-H$,<br>$-C\underline{H_2}-Ph$),<br>4.00−4.67 (3H, m,<br>$-CO-C\underline{H}-S-$, $C_2-H$),<br>5.02 (1H, dd, J=4.5,<br>9.5Hz, $C_4-H$),<br>7.23 (5H, s, $-C_6H_5$),<br>10.00 ($-CO_2H$) |

The compounds shown in Table IV were prepared by the same procedure as described above.

Example 11

(4R)-3-[(Carboxymethylamino)acetyl]-2-(2-hydroxyphenyl)-4-thiazolidinecarboxylic acid (compound 76).

(4R)-3-Chloroacetyl-2-(2-hydroxyphenyl)-4-thiazolidinecarboxylic acid (6 g) was added to a stirred solution of glycine (1.5 g) in N sodium hydroxide (80 ml), and stirred overnight at room temperature. The solution was adjusted to pH 1.5 by 20% hydrochloric acid and washed with ethyl acetate. The aqueous layer was adjusted to pH 3.2, and the separated crystals were collected by filtration to 3.28 g (48.2%) of the titled compound: mp 181—182°C (dec.) (water); $[\alpha]_D^{24}$ +271.2° (c=0.5, MeOH). IR (KBr, $cm^{-1}$): 3400, 3200, 1740, 1672, 1560, 1440, 1380, 1335, 1212, 752, 648, NMR ($K_2CO_3$ in $D_2O$, δ): 3.0—4.3 (6H, m, $C_5$—H, $CO\underline{CH_2}NH\underline{CH_2}CO_2H$), 6.33 and 6.43 (1H, each s, $C_2$—H), 6.6—7.3 (3H, m, arom. H), 7.82 (1H, br. d, J=8Hz, arom. H), 9.0—10.3 (2H, br. s, —OH, —$CO_2H$).

The compounds shown in Table V were prepared by the same procedure as described above.

In EXAMPLEs and TABLEs I, II, III, IV and V, "a" and "b", of compound No. represent diastereoisomers each other. TABLEs I, II, III, IV and V show various compounds and their physical constants including the compounds specified in EXAMPLEs.

TABLE I

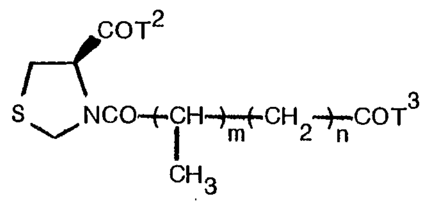

Compound No. 1

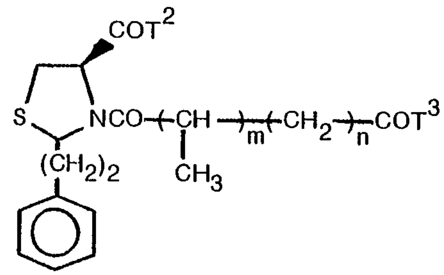

Compound No. 2a and 2b

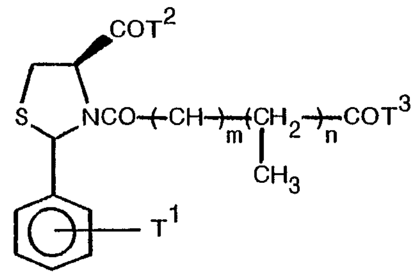

Compound No. 3–32

| Compd.+ No. | $T^1$ | $T^2$ | $T^3$ | m | n | Method of prepn. (Examp. No.) | Yield (%) | mp (°C) (Recrystn. solvent) | $[\alpha]_D$ deg. (c, solv., °C) | IR Spectrum: Sampling method *1 | IR Spectrum: $cm^{-1}$ | Rf value *2 ($SiO_2$) |
|---|---|---|---|---|---|---|---|---|---|---|---|---|
| 1 | | OH | OH | 0 | 6 | 1 | 55 | oil | –84.3 (0.8, MeOH, 26) | C | 1720, 1605, 1420, 1190, 1015, 880 | 0.39 |
| 2a | | OH | OH | 0 | 3 | 5 | 26 | oil | –19.8 (1.1, MeOH, 24) | C | 1733, 1710, 1650, 1600, 1410, 1240, 1040 | 0.60 *3 |
| 2b | | OH | OH | 0 | 3 | 5 | 51 | oil | –113.8 (1.1, MeOH, 24) | C | 1730, 1650, 1610, 1410, 1240, 1042 | 0.55 *3 |
| 3 | 2–OH | OH | OH | 0 | 1 | 1 | 65 | 154.0–154.5 (dec.) ($H_2O$) | +201.4 (0.7, MeOH, 25) | B | 3340, 1725, 1625, 1600, 1460, 1430, 1235, 1100, 915, 770 | 0.25 |
| 4 | 2–OH | OH | OMe | 1 | 1 | 6 | 44 | oil | +161.6 (1.0, MeOH, 25) | A | 3380, 1723, 1624, 1235, 1200, 1174, 764 | 0.51 |
| 5 | 2–OH | OH | OH | 1 | 1 | 7 | 75 | 163–164 dec.) (EtOAc) | +174.1 (1.0, MeOH, 25) | B | 3330, 1730, 1710, 1629, 1280, 1234, 856, 771 | 0.41 |

TABLE I (Continued)

| Compd.[+] No. | $T^1$ | $T^2$ | $T^3$ | m | n | Method of prepn. (Examp. No.) | Yield (%) | mp (°C) (Recrystn. solvent) | $[\alpha]_D$ deg. (c, solv., °C) | IR Spectrum Sampling[*1] method | IR Spectrum $cm^{-1}$ | Rf value[*2] ($SiO_2$) |
|---|---|---|---|---|---|---|---|---|---|---|---|---|
| 6 | 2–OH | OH | OH | 0 | 2 | 1<br>5 | 75 | 190–191 (dec.)<br>(EtOAc–MeOH) | +181.6<br>(1.0, MeOH, 27) | B | 3210, 1720, 1618, 1602, 1245, 1173, 940, 763 | 0.35 |
| 7 | 2–OH | OH | OMe | 0 | 2 | 6 | 83 | 165–166 (dec.)<br>(EtOAc) | +164.5<br>(1.0, MeOH, 25) | A | 3370, 1750, 1693, 1635, 1215, 1165, 755 | 0.47 |
| 8a | 2–OH | OEt | OH | 0 | 2 | 5 | 45 | 181–182<br>(EtOAc) | –2.8<br>(0.5, MeOH, 21) | A | 3310, 1727, 1703, 1637, 1595, 1235, 1190, 745 | 0.55 |
| 8b | 2–OH | OEt | OH | 0 | 2 | 5 | 23 | 116–118<br>(EtOAc) | –311.6<br>(0.5, MeOH, 21) | A | 3370, 1735, 1708, 1635, 1597, 1220, 1180, 760 | 0.55 |
| 9a | 2–OH | OH | NHOH | 0 | 2 | 7 | | 172–173 (dec.)<br>($EtOH-H_2O$) | | A | 3375, 3290, 1720, 1657, 1625, 1590, 1240, 1088, 748 | 0.22 |
| 9b | 2–OH | OH | NHOH | 0 | 2 | 7 | | amorph. | | A | 3220, 1717, 1655, 1625, 1595, 1225, 1092, 752 | 0.33 |
| 10a | 2–OH | OEt | NHOH | 0 | 2 | 8 | | amorph. | | A | 3220, 1727, 1625, 1595, 1200, 1092, 753 | 0.25[*4] |
| 10b | 2–OH | OEt | NHOH | 0 | 2 | 8 | | amorph. | | | | 0.32[*4] |
| 11[*5] | 2–OH | OH | OMe | 1 | 2 | 6 | | amorph. | +55.5<br>(0.8, MeOH, 24) | B | 1738, 1630, 1585, 1310, 1258, 750 | |
| 11a[*5] | 2–OH | OH | OMe | 1 | 2 | 6 | | 205–207 (dec.)<br>(benzene) | +94.6<br>(0.5, MeOH, 23) | B | 3110, 1730, 1625, 1010, 1192, 1121, 758 | |
| 12a | 2–OH | OH | OH | 1 | 2 | 7 | 79 | 168–170 (dec.)<br>(acetone-cyclohexane) | +168.0<br>(0.4, MeOH, 23) | A | 3370, 1718, 1625, 1598, 758 | 0.25[*4] |

TABLE I (Continued)

| Compd.+ No. | $T^1$ | $T^2$ | $T^3$ | m | n | Method of prepn. (Examp. No.) | Yield (%) | mp (°C) (Recrystn. solvent) | $[\alpha]_D$ deg. (c, solv., °C) | IR Spectrum: Sampling*1 method | IR Spectrum: $cm^{-1}$ | Rf value*2 ($SiO_2$) |
|---|---|---|---|---|---|---|---|---|---|---|---|---|
| 12b | 2–OH | OH | OH | 1 | 2 | 7 | | 163–164 (dec.) (acetone-cyclohexane) | +149.2 (0.4, MeOH, 23) | A | 3300, 1720, 1708, 1615, 1598, 1242, 753 | 0.25*4 |
| 13 | 2–OH | OH | OH | 0 | 3 | 5 | 65 | 161–162 (dec.) ($H_2O$) | +153.8 (0.5, MeOH, 24) | B | 3190, 1713, 1632, 1598, 1253, 1098, 943, 760 | 0.38 |
| 14 | 2–OH | OH | OEt | 0 | 3 | 6 | 88 | 157–158 (dec.) (EtOAc-benzene) | +145.6 (1.0, MeOH, 25) | A | 3340, 1725, 1638, 1597, 1218, 1120, 768 | 0.48 |
| 15 | H | OH | OH | 0 | 3 | 5 | 73 | 139–140 (EtOAc–MeOH) | +106.3 (1.0, MeOH, 24) | B | 3170, 1753, 1709, 1631, 1423, 1177, 729 | 0.39 |
| 16 | 4–CN | OH | OH | 0 | 3 | 5 | 59 | 190–191 (EtOAc–MeOH) | +137.7 (1.0, MeOH, 24) | B | 2225, 1710, 1665, 1412, 1258 | 0.31 |
| 17 | 2–OH | OH | OH | 0 | 4 | 1 | 62 | amorph. | +115.6 (1.0, MeOH, 24) | B | 3300, 1700, 1622, 1595, 760, 723 | 0.43 |
| 18 | 2–OH | OH | OH | 0 | 5 | 1 | 60 | 158–159 (dec.) (EtOAc) | +128.6 (0.5, MeOH, 25) | B | 3300, 1710, 1620, 1595, 1280, 1095, 895, 850, 760 | 0.47 |
| 19 | H | OH | OH | 0 | 6 | 1 | 33 | oil | +80.5 (1.0, MeOH, 24) | | | 0.50 |
| 20 | 2–OH | OH | OH | 0 | 6 | 1 | 61 | 155–157 (dec.) (EtOAc) | +134.1 (0.5, MeOH, 27) | B | 3220, 1710, 1620, 1600, 1415, 1235, 1172, 950, 760 | 0.52 |
| 21 | 2–OH | OH | OH | 0 | 7 | 1 | 63 | 153–154 (dec.) (EtOAc) | +70.9 (0.5, MeOH, 26) | B | 3220, 1705, 1620, 1600, 1415, 1235, 1173, 1090, 830, 760 | 0.55 |
| 22 | 3–$NO_2$ | OH | OH | 0 | 7 | 1 | 45 | oil | +72.1 (0.4, MeOH, 27) | C | 1710, 1615, 1525, 1405, 1350, 1095, 735 | 0.56 |

TABLE I (Continued)

| Compd.+ No. | $T^1$ | $T^2$ | $T^3$ | m | n | Method of prepn. (Examp. No.) | Yield (%) | mp (°C) (Recrystn. solvent) | $[\alpha]_D$ deg. (c, solv., °C) | IR Spectrum Sampling method *1 | IR Spectrum $cm^{-1}$ | Rf value *2 ($SiO_2$) |
|---|---|---|---|---|---|---|---|---|---|---|---|---|
| 23 | 3–$NO_2$ | OH | OEt | 0 | 7 | 6 | 79 | oil | +72.8 (1.0, MeOH, 23) | C | 1735, 1663, 1620, 1533, 1352, 1240, 1190, 728 | 0.57 |
| 24 | 2–F | OH | OH | 0 | 7 | 1 | 53 | oil | +69.9 (0.5, MeOH, 23) | C | 1730, 1660, 1625, 1587, 1228, 1043, 756 | 0.57 |
| 25 | 3–F | OH | OH | 0 | 7 | 1 | 50 | oil | +63.4 (0.5, MeOH, 23) | C | 1730, 1655, 1610, 1590, 1243, 1042, 775 | 0.57 |
| 26 | 4–F | OH | OH | 0 | 7 | 1 | | oil | +57.9 (0.8, MeOH, 23) | | | 0.51 *4 |
| 27 | 2–Cl 5–$NO_2$ | OH | OH | 0 | 7 | 1 | 45 | amorph. | *108.3 (0.5, MeOH, 23) | A | 1720, 1660, 1580, 1526, 1240, 1050, 745 | 0.57 |
| 28 | 2–OH | OH | OH | 0 | 8 | 1 | 58 | oil | +100.3 (1.0, MeOH, 24) | C | 1710, 1620, 1600, 1410, 1230, 1090, 850, 760 | 0.58 |
| 29 | 2–OH | OH | OH | 0 | 10 | 1 | 55 | 123–124 (EtOAc-cyclohexane) | +120.4 (0.5, MeOH, 25) | B | 3320, 1705, 1620, 1595, 1410, 1233, 1090, 943, 850, 760 | 0.61 |
| 30 | 3–CN | OH | OH | 0 | 10 | 1 | 56 | oil | +56.4 (0.3, MeOH, 23) | | | 0.56 *4 |
| 31 | 2–OH | OH | OH | 0 | 12 | 1 | 59 | amorph. | +101.4 (1.0, MeOH, 24) | B | 3280, 1700, 1620, 1575, 760, 722 | 0.52 |
| 32 | 3–CN | OH | OH | 0 | 12 | 1 | 43 | oil | +61.7 (0.6, MeOH, 23) | | | 0.53 *4 |

+ a and b represent diastereoisomers of the compound.

*1 A; KBr disk, B; nujol mull, C; neat.

*2 EtOAc–$CHCl_3$–AcOH (10:5:3).

*3 $CHCl_3$–EtOH–AcOH (10:2:1).

*4 EtOAc–$CHCl_3$–AcOH (7:5:1).

*5 Dicyclohexylamine salt.

TABLE II

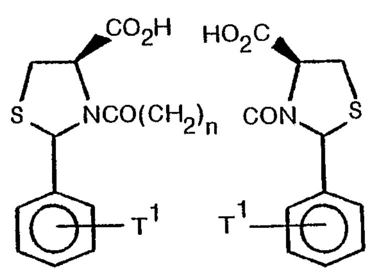

Compound No. 33–37, 39–62

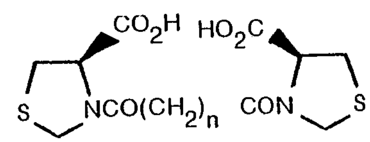

Compound No. 38

| Compd. No. | $T^1$ | n | Method of prepn. (Examp. No.) | Yield (%) | mp (°C) (Recrystn. solvent) | $[\alpha]_D$ deg. (c, solv., °C) | IR Spectrum: Sampling method *1 | IR Spectrum: $cm^{-1}$ | Rf value *2 ($SiO_2$) |
|---|---|---|---|---|---|---|---|---|---|
| 33 | 2–OH | 4 | 2 | 73 | 124–128 (MeOH) | +182.2 (1.0, DMF, 24) | B | 3280, 1726, 1620, 1596, 775 | 0.23 |
| 34 | 2–OH | 5 | 2 | 67 | oil | +106.1 (0.5, MeOH, 26) | C | 1725, 1625, 1600, 1410, 1235, 1095, 1045, 850, 765 | 0.27 |
| 35 | 3–$NO_2$ *5 | 5 | 4 | 69 | 111–113 (dec.) ($H_2O$) | +88.2 (0.5, MeOH, 25) | B | 1635, 1585, 1520, 1355, 1095, 730 | 0.28 |
| 36 | 3–CN | 5 | 3 | 59 | 105–112 ($H_2O$) | +115.0 (1.0, MeOH, 25) | B | 2270, 1735, 1640, 1610, 1195, 790 | 0.33 |
| 37 | 4–CN | 5 | 3 | 52 | amorph. | +148.2 (0.9, MeOH, 25) | B | 2255, 1731, 1655, 1620, 785 | 0.32 |

TABLE II (Continued)

| Compd. No. | $T^1$ | n | Method of prepn. (Examp. No.) | Yield (%) | mp (°C) (Recrystn. solvent) | $[\alpha]_D$ deg. (c, solv., °C) | IR Spectrum Sampling method *1 | IR Spectrum $cm^{-1}$ | Rf value *2 ($SiO_2$) |
|---|---|---|---|---|---|---|---|---|---|
| 38 | | 6 | 2 | 77 | oil | −124.5 (0.5, MeOH, 26) | C | 1720, 1580, 1410, 1180, 1015, 880 | 0.09 |
| 39 | H | 6 | 2 | 79 | amorph. | +97.4 (1,0, MeOH, 24) | B | 1720, 1625, 1585, 732 | 0.42 |
| 40 | 2–OH | 6 | 2 | 86 | amorph. | +123.6 (0.5, MeOH, 27) | B | 1720, 1620, 1600, 1230, 1090, 855, 765 | 0.34 |
| 41 | 3–$NO_2$ | 6 | 2 | 56 | amorph. | +97.5 (0.5, MeOH, 21) | B | 1730, 1650, 1605, 1520, 1345, 1095, 730 | 0.34 |
| 42 | 3–CN | 6 | 2 | 58 | amorph. | +98.3 (0.9, MeOH, 25) | B | 2250, 1730, 1640, 1615, 1200, 790 | 0.38 |
| 43 | 4–CN | 6 | 2 | 41 | amorph. | +130.2 (0.9, MeOH, 25) | B | 2248, 1729, 1650, 1618, 790 | 0.36 |
| 44 | 2–OH | 7 | 2 | 75 | amorph. | +142.7 (0.5, MeOH, 26) | B | 1720, 1620, 1600, 1410, 1230, 1173, 1090, 855, 763 | 0.40 |
| 45 | 2–$NO_2$ | 7 | 2 | 47 | amorph. | +191.2 (0.6, MeOH, 25) | B | 1735, 1655, 1515, 1345, 1190, 730 | 0.38 |
| 46 *6 | 3–$NO_2$ | 7 | 9 | 96 | 61–63 (benzene) | +79.4 (1.0, MeOH, 23) | A | 1740, 1660, 1530, 1350, 1198, 725 | 0.57 |
| 47 | 3–$NO_2$ | 7 | 2 | 82 | amorph. | +96.2 (0.5, MeOH, 27) | B | 1725, 1615, 1520, 1445, 1350, 1095, 730 | 0.41 |
| 48 | 4–$NO_2$ | 7 | 2 | 53 | amorph. | +118.5 (0.5, MeOH, 25) | B | 1730, 1650, 1600, 1510, 1345, 1185, 1110, 735 | 0.48 |

TABLE II (Continued)

| Compd. No. | $T^1$ | n | Method of prepn. (Examp. No.) | Yield (%) | mp (°C) (Recrystn. solvent) | $[\alpha]_D$ deg. (c, solv., °C) | IR Spectrum: Sampling method *1 | IR Spectrum: $cm^{-1}$ | Rf value *2 ($SiO_2$) |
|---|---|---|---|---|---|---|---|---|---|
| 49 | 3–CN | 7 | 3 | 65 | amorph. | +112.1 (1.1, MeOH, 25) | B | 2250, 1729, 1640, 1610, 790 | 0.41 |
| 50 *5 | 2–F | 7 | 4 | 85 | 140–220 (dec.) ($H_2O$) | +117.5 (1.0, MeOH, 24) | A | 1580, 1225, 1173, 758 | 0.50 |
| 51 *5 | 3–F | 7 | 4 | 88 | 195–210 (dec.) ($H_2O$) | +103.9 (0.5, MeOH, 25) | A | 1590, 1238, 1142, 767 | 0.50 |
| 52 | 4–F | 7 | 2 | 76 | oil | +75.8 (1.0, MeOH, 23) | | | 0.39 *3 |
| 53 | 2–Cl 5–$NO_2$ | 7 | 2 | 79 | amorph. | +167.9 (0.5, MeOH, 23) | A | 1725, 1640, 1575, 1520, 1342, 1047, 740 | 0.51 |
| 54 | 2–OH 5–$SO_2NH_2$ | 7 | 2 | 75 | amorph. | +140.9 (0.6, MeOH, 23) | B | 1725, 1620, 1595, 1310, 1150, 930, | 0.42 *4 |
| 55 | 2–OH | 8 | 2 | 68 | amorph. | +122.1 (1.0, MeOH, 24) | B | 3300, 1730, 1628, 1575, 767, 725 | 0.45 |
| 56 | 3–CN | 8 | 2 | 47 | amorph. | +104.6 (1.0, MeOH, 25) | B | 2245, 1726, 1630, 1610, 790 | 0.37 |
| 57 | 3–$NO_2$ | 8 | 2 | 84 | amorph. | +102.2 (0.5, MeOH, 25) | A | 1735, 1620, 1523, 1190, 728 | 0.47 |
| 58 *5 | 3–$NO_2$ | 8 | 4 | 74 | amorph. | +93.9 (0.5, MeOH, 23) | A | 1597, 1520, 1269, 1096, 723 | |
| 59 | 2–OH | 10 | 2 | 61 | 99–100.5 (dec.) (EtOAc-benzene) | +124.7 (0.5, MeOH, 27) | B | 3300, 1740, 1620, 1600, 1565, 1230, 1160, 1090, 895, 770 | 0.49 |

TABLE II (Continued)

| Compd. No. | $T^1$ | n | Method of prepn. (Examp. No.) | Yield (%) | mp (°C) (Recrystn. solvent) | $[\alpha]_D$ deg. (c, solv., °C) | IR Spectrum: Sampling method *1 | IR Spectrum: $cm^{-1}$ | Rf value *2 ($SiO_2$) |
|---|---|---|---|---|---|---|---|---|---|
| 60 *5 | 3–CN | 10 | 4 | 63 | 190–195 ($H_2O$) | +109.3 (0.5, $H_2O$, 23) | B | 3400, 2240, 1640, 1600, 1208, 778, 720 | 0.49 |
| 61 | 2–OH | 12 | 2 | 66 | amorph. | +69.5 (1.0, MeOH, 24) | B | 3300, 1728, 1630, 1590, 762, 725 | 0.45 |
| 62 *5 | 3–CH | 12 | 4 | 52 | amorph. | +104.2 (0.5, MeOH, 23) | B | 3400, 2225, 1605, 1320, 1207, 775, 720 | 0.46 *3 |

*1 A; KBr disk, B; nujol mull, C; neat.

*2 EtOAc–$CHCl_3$–AcOH (10:5:3).

*3 EtOAc–$CHCl_3$–AcOH (7:5:1).

*4 $CHCl_3$–MeOH–AcOH (3:1:1).

*5 Disodium salt.

*6 Dimethyl ester.

TABLE III

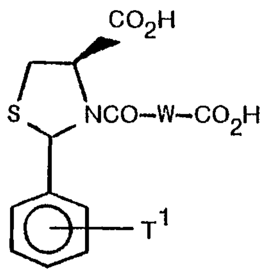

Compound No. 63–68

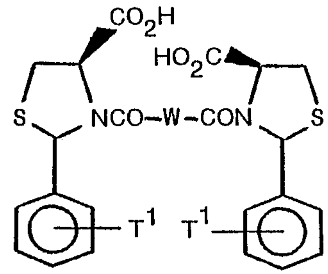

Compound No. 69–71

| Compd. No. | $T^1$ | W | Method of prepn. (Examp. No.) | Yield (%) | mp (°C) (Recrystn. solvent) | $[\alpha]_D$ deg. (c, solv., °C) | IR Spectrum Sampling method *1 | cm$^{-1}$ | Rf value ($SiO_2$) |
|---|---|---|---|---|---|---|---|---|---|
| 63 | 2–OH | $-CH_2COCH(COCH_3)-$ | 5 | 31 | amorph. | +149.2 (1.2, MeOH, 25) | B | 1743, 1720, 1630, 1600, 1238 | 0.38 *3 |
| 64 | 2–OH | $-CH_2-O-CH_2-$ | 1 | 35 | amorph. | +138.6 (1.1, MeOH, 25) | A | 3300, 1726, 1640, 1453, 1234, 1142 | 0.24 *4 |
| 65 | 3–$NO_2$ | 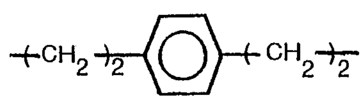 | 1 | 36 | amorph. | +81.7 (0.9, MeOH, 24) | A | 3400, 1702, 1618, 1525, 1400, 1347 | 0.55 *3 |
| 66 | 2–OH | $-(CH_2)_2-O-(CH_2)_2-$ | 1 | 33 | 136–137 (EtOAc) | +147.6 (0.5, MeOH, 25) | B | 3320, 1750, 1710, 1625, 1595, 1235, 1110, 855, 770 | 0.28 |

TABLE III (Continued)

| Compd. No. | $T^1$ | W | Method of prepn. (Examp. No.) | Yield (%) | mp (°C) (Recrystn. solvent) | $[\alpha]_D$ deg. (c. solv., °C) | IR Spectrum Sampling method *1 | IR Spectrum $cm^{-1}$ | Rf value *2 ($SiO_2$) |
|---|---|---|---|---|---|---|---|---|---|
| 67 | 2–OH | $-(CH_2)_2-S-(CH_2)_2-S-(CH_2)_2-$ | 1 | 35 | amorph. | +78.1 (1.0, MeOH, 24) | B | 3300, 1715, 1627, 1590, 760 | 0.31 |
| 68 | 3–$NO_2$ | 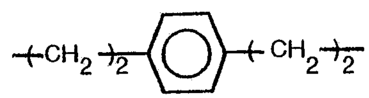 | 2 | 44 | amorph. | +106.9 (1.1, MeOH, 24) | A | 3425, 1730, 1640, 1525, 1400, 1350 | 0.38 *3 |
| 69 | 2–OH | $-(CH_2)_2-O-(CH_2)_2-$ | 2 | 47 | amorph. | +83.0 (0.5, MeOH, 26) | B | 1720, 1625, 1600, 1230, 1090, 850, 760 | 0.15 |
| 70 | 2–OH | $-(CH_2)_2-S-(CH_2)_2-$ | 2 | 53 | amorph. | +129.3 (0.5, MeOH, 27) | B | 1720, 1620, 1600, 1420, 1230, 1093, 852, 763 | 0.30 |

*1 A; KBr disk, B; nujol mull.

*2 EtOAc–$CHCl_3$–AcOH (10:5:3).

*3 EtOAc–EtOH–AcOH (40:1:1).

*4 $CHCl_3$–EtOH–AcOH (10:2:1).

TABLE IV

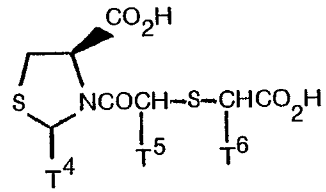

Compound No. 71–75

| Compd.[+] No. | $T^4$ | T5 | T6 | Method of prepn. (Examp. No.) | Yield (%) | mp (°C) (Recrystn. solvent) | $[\alpha]_D$ deg. (c, solv., °C) | IR Spectrum: Sampling method[*1] | IR Spectrum: $cm^{-1}$ | Rf value[*2] ($SiO_2$) |
|---|---|---|---|---|---|---|---|---|---|---|
| 71a | H | $CH_3$ | Ph | 10 | 38 | 151–153 (EtOAc) | +8.6 (1.0, MeOH, 23) | A | 3030, 1737, 1720, 1615, 1413, 1215, 1150, 717 | 0.26[*3] |
| 71b | H | $CH_3$ | Ph | 10 | 49 | oil | −161.5 (1.0, MeOH, 23) | C | 1735, 1623, 1413, 1243, 1170, 1043, 699 | 0.22[*3] |
| 72 | 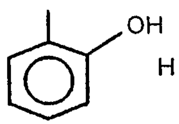 | H | $CH_2CH_2Ph$ | 10 | 81 | amorph. | +122.1 (1.2, MeOH, 25) | A | 1720–1710, 1625, 1600, 1400, 1235, 752, 698 | 0.74 |
| 73 | H | $CH_3$ | $CH_2CH_2Ph$ | 10 | 52 | amorph. | −97.9 (1.1, MeOH, 25) | A | 1720, 1620, 1415, 750, 700 | 0.65 |
| 74a | H | $CH_2Ph$ | H | 10 | 37 | oil | −52.2 (1.2, MeOH, 25) | C | 1720, 1620, 1422, 1217, 756 | 0.13[*4] |
| 74b | H | $CH_2Ph$ | H | 10 | 46 | oil | −60.4 (1.0, MeOH, 25) | C | 1722, 1620, 1420, 1215, 755 | 0.13[*4] |
| 75 | H | $CH_2CH_2Ph$ | H | 10 | 84 | oil | −61.2 (1.3, MeOH, 24) | C | 1735, 1630, 1615, 1420, 1242, 1172, 1043, 702 | 0.66 |

+ a and b represent diastereoisomers of the compound.
*1 A; KBr disk, C; neat.
*2 EtOAc–$CHCl_3$–AcOH (10:5:3).
*3 Benzene–EtOAc–EtOH–AcOH (14:14:2:1).
*4 Benzene–EtOAc–AcOH (25:25:1).

TABLE V

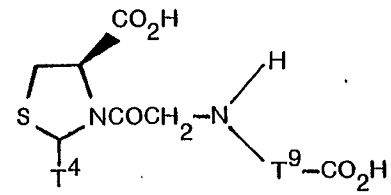

Compound No. 76–80

| Compd.+ No. | $T^4$ | $T^9$ | Method of prepn. (Examp. No.) | Yield (%) | mp (°C) (Recrystn. solvent) | $[\alpha]_D$ deg. (c, solv., °C) | IR Spectrum Sampling method*1 | IR Spectrum $cm^{-1}$ | Rf value ($SiO_2$) |
|---|---|---|---|---|---|---|---|---|---|
| 76 | 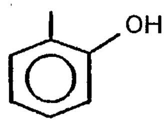 | $-CH_2-$ | 11 | 48.2 | 181–182 (dec.) ($H_2O$) | +271.2 (0.5, N NaOH, 24) | A | 3400, 3200, 1740, 1672, 1560, 1440, 1380, 1335, 1210, 752 | 0.25 *2 |
| 77 | 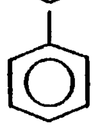 | $-CH_2-$ | 11 | 32.8 | 150–155 ($H_2O$) | +94.7 (0.5, N NaOH, 23) | B | 3420, 3210, 1650, 1240, 839, 790 | 0.45 *3 |
| 78 | 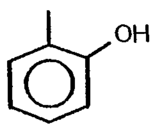 | 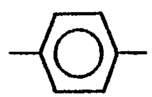 | 11 | 44.8 | 150–153 (dec.) (EtOH–ether) | +86.5 (0.4, MeOH, 26) | A | 3370–2900, 1655, 1602, 1175 | 0.74 *4 |
| 79 | 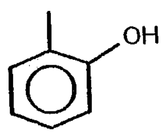 | 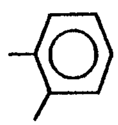 | 11 | 50.3 | 172–173 (dec.) (EtOAc) | +78.9 (0.8, MeOH, 25) | A | 3350, 1720, 1670, 1644, 1236, 744 | 0.69 *4 |
| 80 | 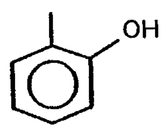 | $CH_2CH_2Ph$ <br> \| <br> $-CH-$ | 11 | 27.2 | 174–175 (dec.) ($H_2O$) | | A | 3400, 1720, 1660, 1610, 1492, 1452, 1240, 752 700 | |

*1 A; KBr disk, B; nujol mull, C; Neat, D; liquid cell ($CHCl_3$).
*2 n-BuOH–AcOH–$H_2O$ (4:2:1).
*3 n-BuOH–AcOH–$H_2O$ (4:1:2).
*4 EtOAc–$CHCl_3$–AcOH (10:5:3).

*Pharmacological Test*

It has been known that aldose reductase participates in diabetic cataract which is one of the diabetic complications and that appearance is retarded or depressed by inhibition of the aldose reductase [Acta Societatis Ophthalmologicae Japonicae, *80*, 1362 (1987)]. The following method is used for the present test.

(Method)

Aldose reductase is purified from rat lenses according to the method of Hoyman et al. [J. Biol. Chem., *240*, 877 (1965)]. Action of the compounds (I) of this invention is evaluated by measurement of optical density according to the J. H. Kinoshita's method [Invest. Ophthal., *13*, 713 (1974)]. The reaction mixture for the measurement of the aldose reductase activity is 3.0 ml [0.007 M phosphate buffer solution (pH 6.2), 0.46 M lithium sulfate, $5 \times 10^{-}$ M NADPH, $4 \times 10^{-4}$ M DL-glyceraldehyde, 10U aldose reductase, $10^{-4}$ to $10^{-10}$ M the compounds (I)] as total volume, and the absorbance thereof is measured at 340 nm.

(Result)

Table VI shows that the compounds (I) of this invention have a strong aldose reductase inhibition effect.

TABLE VI

Inhibitory Activity of the Thiazolidine Compounds against Aldose Reductase

| Compd. No. | $IC_{50}$(M)*1 |
|---|---|
| 22 | $8.2 \times 10^{-10}$ |
| 23 | $1.1 \times 10^{-8}$ |
| 47 | $1.6 \times 10^{-10}$ |
| 56 | $1.7 \times 10^{-9}$ |
| 57 | $5.4 \times 10^{-9}$ |
| control *2 | $1.0 \times 10^{-7}$ |

*1 Molar concentration of a compound producing 50% inhibition of aldose reductase.
*2 Quercitrin: referred to Acta Societatis Ophthalmologicae Japonicae, *80*, 1369—1370 (1976).

*Toxicity Test*

The acute toxicity of compounds 47 and 56 is 1000 ~ 1500 mg/kg.

(Experimental animals)

The male ddy-std. strain mice (4 weeks of age, weighing 19—21 g) were placed in a breeding room of constant temperature and humidity (23 ± 1°C, 55 ± 5%) and fed freely pellet diet (CE—2, Clea Japan, Inc.) and water ad. libitum for a week. The mice showing the normal growth were selected for the experiment.

(Method of administration)

Test compounds are dissolved in distilled water and administered (i.v.) in a dose of 0.5 ml/20 g body weight.

It is found in the above pharmacological and toxicity test that the compounds (I) of this invention are useful as drugs for therapy or prophylaxis of the diabetic complications.

In case the compounds are used for preventing or relieving diabetic complications, the dosage forms are tablet, capsule, granule, powder, suppository, injection, ophthalmic solution, ophthalmic ointment, etc. These preparations can also contain general excipients.

The dose is adjusted depending on sympton, dosage form, etc. But, usual daily dosage is 1 to 5000 mg, preferably 10 to 1000 mg, in one or a few divided doses.

*Examples of Formulation*
(1) Oral drug
(a) tablet

| | |
|---|---|
| compound 13 | 50 mg |
| lactose | 120 mg |
| crystalline cellulose | 60 mg |
| calcium carboxymethylcellulose | 7 mg |
| magnesium stearate | 3 mg |
| Total | 240 mg |

| | |
|---|---|
| compound 22 | 100 mg |
| lactose | 95 mg |
| crystalline cellulose | 45 mg |
| calcium carboxymethylcellulose | 7 mg |
| magnesium stearate | 3 mg |
| Total | 240 mg |

| | |
|---|---|
| compound 23 | 150 mg |
| lactose | 60 mg |
| crystalline cellulose | 30 mg |
| calcium carboxymethylcellulose | 7 mg |
| magnesium stearate | 3 mg |
| Total | 250 mg |

| | |
|---|---|
| compound 56 | 150 mg |
| lactose | 60 mg |
| crystalline cellulose | 30 mg |
| calcium carboxymethylcellulose | 7 mg |
| magnesium stearate | 3 mg |
| Total | 250 mg |

| | |
|---|---|
| compound 73 | 150 mg |
| lactose | 60 mg |
| crystalline cellulose | 30 mg |
| calcium carboxymethylcellulose | 7 mg |
| magnesium stearate | 3 mg |
| Total | 250 mg |

The tablets may be treated with common film-coating and further sugar-coating.

(b) granule

| | |
|---|---|
| compound 13 | 30 mg |
| polyvinylpyrrolidone | 25 mg |
| lactose | 385 mg |
| hydroxypropylcellulose | 50 mg |
| talc | 10 mg |
| Total | 500 mg |

| | |
|---|---|
| compound 22 | 30 mg |
| polyvinylpyrrolidone | 25 mg |
| lactose | 385 mg |
| hydroxypropylcellulose | 50 mg |
| talc | 10 mg |
| Total | 500 mg |

(c) powder

| | |
|---|---|
| compound 13 | 250 mg |
| lactose | 240 mg |
| starch | 480 mg |
| colloidal silica | 30 mg |
| Total | 1000 mg |

| | |
|---|---|
| compound 65 | 300 mg |
| lactose | 230 mg |
| starch | 440 mg |
| colloidal silica | 30 mg |
| Total | 1000 mg |

(d) capsule

| | |
|---|---|
| compound 13 | 50 mg |
| lactose | 102 mg |
| crystalline cellulose | 36 mg |
| colloidal silica | 2 mg |
| Total | 190 mg |

| | |
|---|---|
| compound 23 | 100 mg |
| lactose | 52 mg |
| crystalline cellulose | 36 mg |
| colloidal silica | 2 mg |
| Total | 190 mg |

| | |
|---|---|
| Compound 73 | 200 mg |
| glycerin | 179.98 mg |
| butyl p-hydroxybenzoate | 0.02 mg |
| Total | 380 mg |

| | |
|---|---|
| compound 76 | 30 mg |
| glycerin | 349.98 mg |
| butyl p-hydroxybenzoate | 0.02 mg |
| Total | 380 mg |

(2) Injection

(a) 1 to 30 mg of compound 9b is contained in 1 ml of the aqueous solution (pH 6.5—7.0).

(b) 1 to 30 mg of compound 73 is contained in 1 ml of the aqueous solution (pH 6.5—7.0).

(3) Ophthalmic solution

The following composition is contained in 5 ml of the aqueous solution (pH 6.0).

| | |
|---|---|
| Compound 23 | 50 mg |
| propyl p-hydroxybenzoate | 0.7 mg |
| methyl p-hydroxybenzoate | 1.3 mg |
| sodium hydroxide | proper quantity |

(4) Ophthalmic ointment

The following composition is contained in 1 g.

| | |
|---|---|
| compound 22 | 20 mg |
| white petrolatum | 889.8 mg |
| mineral oil | 100 mg |
| butyl p-hydroxybenzoate | 0.2 mg |

(5) Suppository

The following composition is contained in 1 g.

| | |
|---|---|
| compound 47 | 50 mg |
| polyethylene glycol 1000 | 800 mg |
| polyethylene glycol 4000 | 150 mg |

**Claims for the Contracting States: BE CH DE FR GB IT LI LU NL SE**

1. A compound of the formula (I)

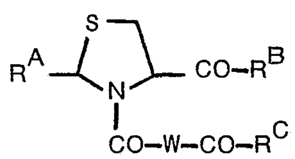

(I)

wherein

$R^A$ is $R^a$ or $R^b$;

$R^B$ and $R^C$ each is $R^c$;

W is 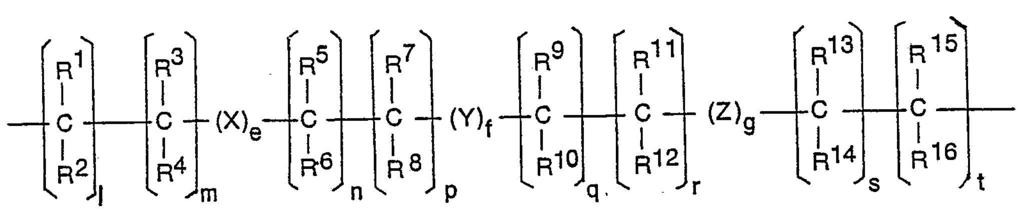

wherein

X, Y and Z are each

$-CH_2-$, 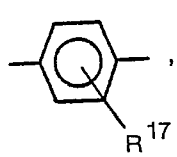 , 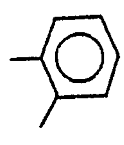 ,

$-O-$, $-CO-$, $-S-$, $-SO-$, $-SO_2-$, 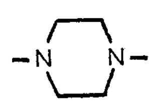 or 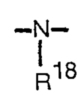 ;

whereby, when any of X, Y and Z is

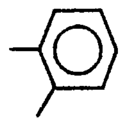 $R^A$ is 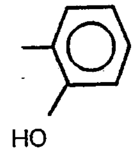 ,

$R^B$ and $R^C$ both are hydroxy and W is represented by

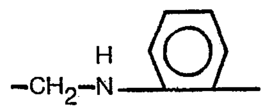 ;

e, f and g are each 0 or 1;
l, m, n, p, q, r, s and t are each 0, 1, 2 or 3, but (l+m) is not 0;
$R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$ and $R^{18}$ are each $R^d$;

when W is 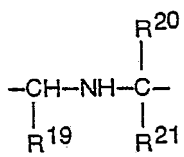 or 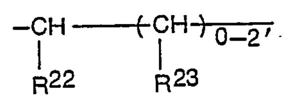

wherein
$R^{19}$, $R^{20}$, $R^{21}$, $R^{22}$ and $R^{23}$ are each $R^d$, $R^A$ is $R^b$ when X is S and e is 1, (n+p) is not 0;
when X is S, e is 1 and

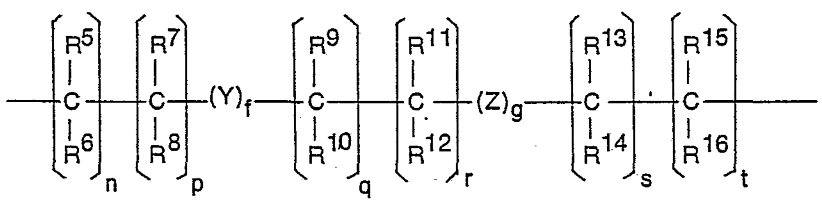

is lower alkylene $R^C$ is not hydroxy;
$R^a$ is hydrogen or ($C_1$—$C_4$) alkyl;
$R^b$ is selected from
(a) (i) benzyl or phenylethyl and
(ii) benzyl or phenylethyl substituted by at least one substituent selected from methyl, hydroxy, methoxy, halogen, nitro and cyano, and
(b) phenyl and phenyl substituted by at least one substituent selected from methyl, hydroxy, methoxy, benzyloxy, acetyloxy, halogen, nitro, cyano, amino, dimethylamino, carboxy, sulfamoyl, difluoromethoxy and dipropylaminosulfonyl;
$R^c$ is selected from
(a) hydroxy, ($C_1$—$C_4$) alkoxy, amino, hydroxyamino and benzyloxy, and

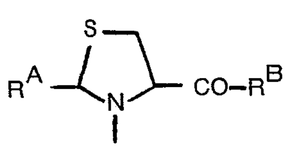 ; (b)

$R^d$ is selected from
(a) hydrogen, ($C_1$—$C_6$) alkyl, benzyl, phenylethyl, acetyl, hydroxy, carboxy and amino, and
(b) phenyl and phenyl substituted by at least one substituent selected from methyl, methoxy, hydroxy, carboxy, amino, halogen, nitro and cyano,
and salts thereof.

2. A compound of claim 1 with the following formula

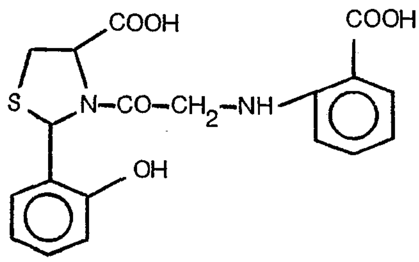

3. A compound of claim 1 wherein $R^a$ is hydrogen.

4. A compound of claim 1 wherein $R^b$ is 2-phenylethyl, phenyl, 2-fluorophenyl, 3-fluorophenyl, 4-fluorophenyl, 2-nitrophenyl, 3-nitrophenyl, 4-nitrophenyl, 2-chloro-5-nitrophenyl, 2-hydroxyphenyl, 3-cyanophenyl, 4-cyanophenyl, 2-hydroxy-5-sulfamoylphenyl.

5. A compound of claim 1 wherein $R^c$ is hydroxy, methoxy, ethoxy, hydroxyamino or

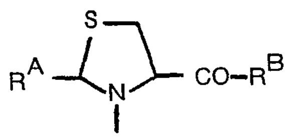 ;

6. A compound of claim 1 wherein $R^d$ is hydrogen, methyl, 2-phenylethyl or phenyl.

7. A compound of claim 1 wherein W is $—CH(CH_3)—CH_2—$, $—CH(CH_3)—(CH_2)_2—$, $—CH_2)_{1-8}$, $—(CH_2)_{10}—$ or $—(CH_2)_{12}—$.

8. A compound of claim 1 wherein W is $—CH_2COCH(COCH_3)—$.

9. A compound of claim 1 wherein W is $—CH_2—O—CH_2—$, $—(CH_2)_2—O—(CH_2)_2—$, $—(CH_2)_2—S—(CH_2)_2—$ or $—(CH_2)_2—S—(CH_2)_2—S—(CH_2)_2—$.

10. A compound of claim 1 wherein W is

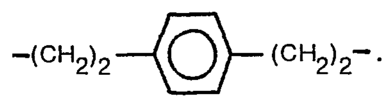

11. A compound of claim 1 wherein W is $—CH(CH_3)—S—CH(Ph)—$, $—CH_2—S—CH(CH_2CH_2Ph)—$, $—CH(CH_3)—S—CH(CH_2CH_2Ph)—$, $—CH(CH_2Ph)—S—CH_2—$, or $—CH(CH_2CH_2Ph)—S—CH_2—$.

12. A compound of claim 1 wherein W is

$-CH_2NHCH_2-$, 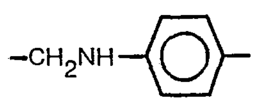 or $-CH_2NH-CH(CH_2CH_2Ph)-$.

13. A compound of claim 1 which is (4R) - 3 - [8 - (ethoxycarbonyl) - octanoyl] - 2 - (3 - nitrophenyl) - 4 - thiazolidinecarboxylic acid.

14. A compound of claim 1 which is (4R,4'R) - 3,3' - (nonanedioyl)bis[2 - (3 - nitrophenyl) - 4 - thiazolidinecarboxylic acid methyl ester].

15. A compound of claim 1 which is (4R) - 3 - (11 - carboxyundecanoyl) - 2 - (3 - cyanophenyl) - 4 - thiazolidinecarboxylic acid.

16. A compound of claim 1 which is (4R,4'R) - 3,3' - (decanedioyl)bis[2 - (3 - cyanophenyl) - 4 - thiazolidinecarboxylic acid].

17. A compound of claim 1 which is (4R,4'R) - 3,3' - (dodecanedioyl)bis[2 - (3 - cyanophenyl) - 4 - thiazolidinecarboxylic acid].

18. A compound of claim 1 which is (4R) - 3 - (8 - carboxyoctanoyl) - 2 - (3 - nitrophenyl) - 4 - thiazolidinecarboxylic acid.

19. A compound of claim 1 which is (4R,4'R) - 3,3' - (nonanedioyl)bis[2 - (3 - nitrophenyl) - 4 - thiazolidinecarboxylic acid].

20. A compound of claim 1 which is (4R) - 3 - (7 - carboxyheptanoyl) - 2 - (2 - hydroxyphenyl) - 4 - thiazolidinecarboxylic acid.

21. A compound of claim 1 which is (4R) - 3 - [[(1 - carboxy - 3 - phenylpropyl)amino]acetyl] - 2 - (2 - hydroxyphenyl) - 4 - thiazolidinecarboxylic acid.

22. A compound of claim 1 which is (4R) - 3 - [[(1 - carboxy - 3 - phenylpropyl)thio]acetyl] - 2 - (2 - hydroxyphenyl) - 4 - thiazolidinecarboxylic acid.

23. A compound of claim 1 which is (4R) - 3 - (4 - carboxybutanoyl) - 2 - (2 - hydroxyphenyl) - 4 - thiazolidinecarboxylic acid.

24. A process for preparing a compound of the formula (I) according to patent claim 1 and salts thereof which comprises

(i) reacting a compound of the formula [II]

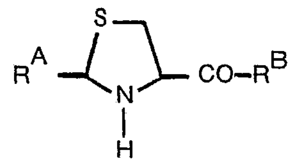

(II)

wherein $R^A$ and $R^B$ may include suitable protection of any reactive groups with the reactive derivative of a carboxylic acid of the formula [III]

$$HOOC—W—CO—R^C \qquad [III]$$

wherein $R^C$ and W may include suitable protection of any reactive groups, followed by removal of protective groups, if necessary, to yield a compound of the formula [I];

(ii) reacting a compound of the formula [II] with the reactive derivative of carboxylic acid of the formula [IV]

$$HOOC—W^1—L \qquad [IV]$$

wherein $W^1$ is 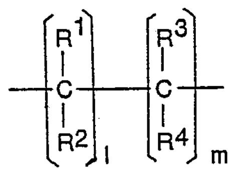 ,

and may include suitable protection of any reactive groups, and L is a leaving group to yield a compound of the formula [V]

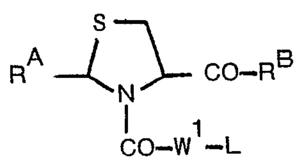

(V)

and then reacting a compound of the formula [V] with a compound of the formula [VI]

$$(H)(X)_eW^2—(Y)_f—W^3—(Z)_g—W^4—CO—R^C \qquad [VI]$$

wherein

$W^2$ is 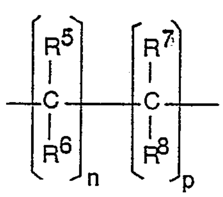 , $W^3$ is 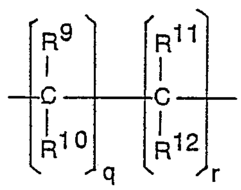 ; $W^4$ is 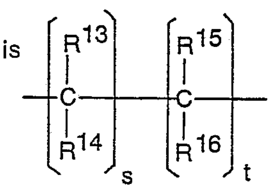

and $W^2$, $W^3$, $W^4$, X, Y, Z and $R^C$ may include suitable protection of any reactive groups, followed by removal of protective groups, if necessary, to yield a compound of the formula [I];

(iii) reacting a compound of the formula [II] with the reactive derivative of carboxylic acid of the formula [VII]

$$HOOC—W^1—(X)_e—W^2—L \qquad [VII],$$

and then with a compound of the formula [VIII]

$$(H)(Y)_f—W^3—(Z)_g—W^4—CO—R^C \qquad [VIII]$$

by the same method as (ii) above to yield a compound of the formula [I];

(iv) reacting a compound of the formula [II] with the reactive derivative of carboxylic acid of the formula [IX]

$$HOOC—W^1—(X)_eW^2—(Y)_f—W^3—L \quad [IX],$$

and then with a compound of the formula [X]

$$(H)(Z)_g—W^4—CO—R^C \quad [X]$$

by the same method as (ii) above to yield a compound of the formula [I];

(v) reacting a compound of the formula [II] with the reactive derivative of carboxylic acid of the formula [XI]

$$HOOC—W^1—(X)_e(H) \quad [XI],$$

and then with a compound of the formula [XII]

$$L—W^2—(Y)_f—W^3—(Z)_g—W^4—CO—R^C \quad [XII]$$

by the same method as (ii) above to yield a compound of the formula [I];

(vi) reacting a compound of the formula [II] with the reactive derivative of carboxylic acid of the formula [XIII]

$$HOOC—W^1—(X)_e—W^2—(Y)_f(H) \quad [XIII],$$

and then with a compound of the formula [XIV]

$$L—W^3—(Z)_g—W^4—CO—R^C \quad [XIV]$$

by the same method as (ii) above to yield a compound of the formula [I], or

(vii) reacting a compound of the formula [II] with the reactive derivative of carboxylic acid of the formula [XV]

$$HOOC—W^1—(X)_e—W^2—(Y)_f—W^3—(Z)_g(H) \quad [XV],$$

and then with a compound of the formula [XVI]

$$L—W^4—CO—R^C \quad [XVI]$$

by the same method as (ii) above to yield a compound of the formula [I]; furthermore converting $R^B$, $R^C$, X, Y and Z to other functional groups by the general methods, if desired, to obtain a desired compound of the formula [I].

25. A pharmaceutical composition comprising a compound of the formula (I) according to patent claim 1 or salts thereof.

26. A composition according to patent claim 25 comprising a compound of the formula (I) or salts thereof in an amouint sufficient to prevent or relieve diabetes mellitus associated complications consisting of cataracts, neuropathy, nephropathy and retinopathy, and pharmaceutically acceptable excipient(s).

27. A compound according to claim 1 to 23 for use in a method for therapy or prophylaxis.

26. Use of a compound according to claim 1 to 23 in a process for producing pharmaceutical compositions.

**Claims for the Contracting State: AT**

1. A process for preparing a compond of the formula (I)

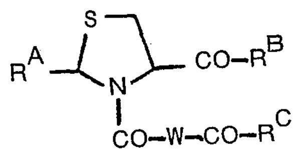

(I)

wherein

$R^A$ is $R^a$ or $R^b$;

$R^B$ and $R^C$ each is $R^C$;

W is 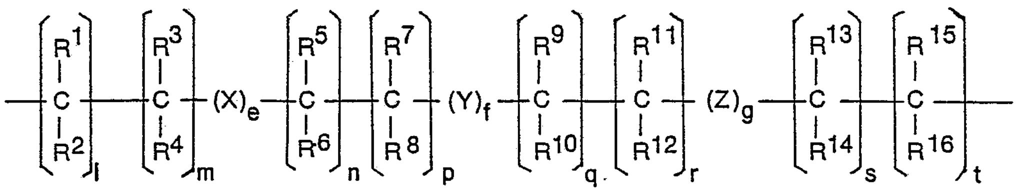

wherein

X, Y and Z are each

$-CH_2-$, 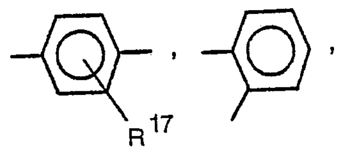,

$-O-$, $-CO-$, $-S-$, $-SO-$, $-SO_2-$, 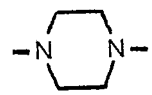 or 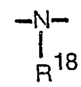 ;

whereby, when any of X, Y and Z is

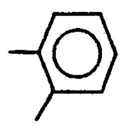 $R^A$ is 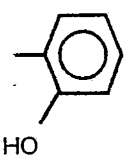,

$R^B$ and $R^C$ both are hydroxy and W is represented by

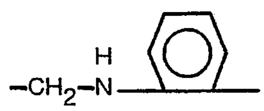 ;

e, f and g are each 0 or 1;
l, m, n, p, q, r, s and t are each 0, 1, 2 or 3, but (l+m) is not 0;
$R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$ and $R^{18}$ are each $R^d$;

when W is 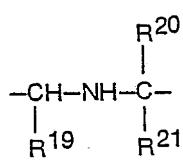 or 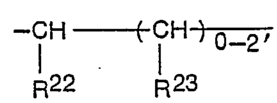

wherein
$R^{19}$, $R^{20}$, $R^{21}$, $R^{22}$ and $R^{23}$ are each $R^d$, $R^A$ is $R^b$ when X is S and e is 1, (n+p) is not 0;
when X is S, e is 1 and

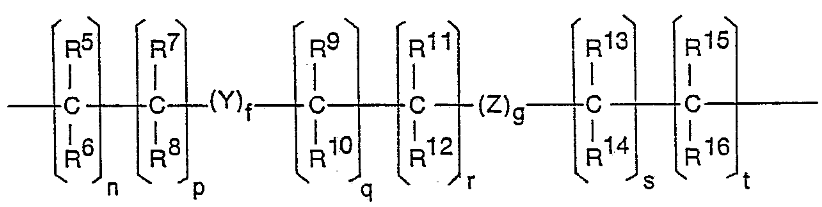

is lower alkylene $R^C$ is not hydroxy;
$R^a$ is hydrogen or ($C_1$—$C_4$) alkyl;
$R^b$ is selected from

(a) (i) benzyl or phenylethyl and
(ii) benzyl or phenylethyl substituted by at least one substituent selected from methyl, hydroxy, methoxy, halogen, nitro and cyano, and
(b) phenyl and phenyl substituted by at least one substituent selected from methyl, hydroxy, methoxy, benzyloxy, acetyloxy, halogen, nitro, cyano, amino, dimethylamino, carboxy, sulfamoyl, difluoromethoxy and dipropylaminosulfonyl;
$R^c$ is selected from
(a) hydroxy, ($C_1$—$C_4$) alkoxy, amino, hydroxyamino and benzyloxy, and

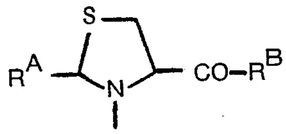 ; (b)

$R^d$ is selected from
(a) hydrogen, ($C_1$—$C_6$) alkyl, benzyl, phenylethyl, acetyl, hydroxy, carboxy and amino, and
(b) phenyl and phenyl substituted by at least one substituent selected from methyl, methoxy, hydroxy, carboxy, amino, halogen, nitro and cyano,
and salts thereof.
which comprises
(i) reacting a compound of the formula [II]

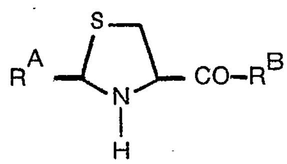 (II)

wherein $R^A$ and $R^B$ may include suitable protection of any reactive groups with the reactive derivative of a carboxylic acid of the formula [III]

HOOC—W—CO—$R^C$ [III]

wherein $R^C$ and W may include suitable protection of any reactive groups, followed by removal of protective groups, if necessary, to yield a compound of the formula [I];
(ii) reacting a compound of the formula [II] with the reactive derivative of carboxylic acid of the formula [IV]

HOOC—$W^1$—L [IV]

wherein

$W^1$ is 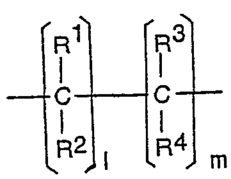 ,

and may include suitable protection of any reactive groups, and L is a leaving group to yield a compound of the formula [V]

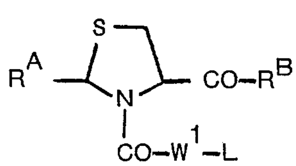 (V)

and then reacting a compound of the formula [V] with a compound of the formula [VI]

$(H)(X)_eW^2—(Y)_f—W^3—(Z)_g—W^4—CO—R^C$ [VI]

wherein

$W^2$ is 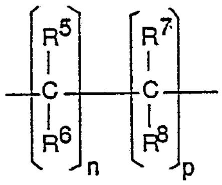 , $W^3$ is 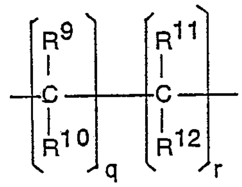 , $W^4$ is 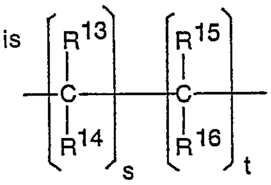

and $W^2$, $W^3$, $W^4$, X, Y, Z and $R^C$ may include suitable protection of any reactive groups, followed by removal of protective groups, if necessary, to yield a compound of the formula [I];

(iii) reacting a compound of the formula [II] with the reactive derivative of carboxylic acid of the formula [VII]

$$HOOC—W^1—(X)_e—W^2—L \qquad [VII],$$

and then with a compound of the formula [VIII]

$$(H)(Y)_f—W^3—(Z)_g—W^4—CO—R^C \qquad [VIII]$$

by the same method as (ii) above to yield a compound of the formula (I);

(iv) reacting a compound of the formula [II] with the reactive derivative of carboxylic acid of the formula [IX]

$$HOOC—W^1—(X)_eW^2—(Y)_f—W^3—L \qquad [IX],$$

and then with a compound of the formula [X]

$$(H)(Z)_g—W^4—CO—R^C \qquad [X]$$

by the same method as (ii) above to yield a compound of the formula [I];

(v) reacting a compound of the formula [II] with the reactive derivative of carboxylic acid of the formula [XI]

$$HOOC—W^1—(X)_e(H) \qquad [XI],$$

and then with a compound of the formula [XII]

$$L—W^2—(Y)_f—W^3—(Z)_g—W^4—CO—R^C \qquad [XII]$$

by the same method as (ii) above to yield a compound of the formula [I];

(vi) reacting a compound of the formula [II] with the reactive derivative of carboxylic acid of the formula [XIII]

$$HOOC—W^1—(X)_e—W^2—(Y)_f(H) \qquad [XIII],$$

and then with a compound of the formula [XIV]

$$L—W^3—(Z)_g—W^4—CO—R^C \qquad [XIV]$$

by the same method as (ii) above to yield a compound of the formula [I], or

(vii) reacting a compound of the formula [II] with the reactive derivative of carboxylic acid of the formula [XV]

$$HOOC—W^1—(X)_e—W^2—(Y)_f—W^3—(Z)_g(H) \qquad [XV],$$

and then with a compound of the formula [XVI]

$$L—W^4—CO—R^C \qquad [XVI]$$

by the same method as (ii) above to yield a compound of the formula [I];

furthermore converting $R^B$, $R^C$, X, Y and Z to other functional groups by the general methods, if desired, to obtain a desired compound of the formula [I].

2. A process according to claim 1 characterized in that a compound with the following formula

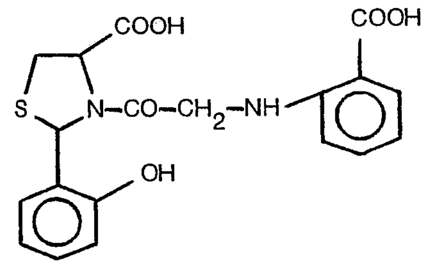

is prepared.

3. A process according to claim 1 characterized in that a compound of formula (I) is prepared wherein $R^a$ is hydrogen.

4. A process according to claim 1 characterized in that a compound of formula (I) is prepared wherein $R^b$ is 2-phenylethyl, phenyl, 2-fluorophenyl, 3-fluorophenyl, 4-fluorophenyl, 2-nitrophenyl, 3-nitrophenyl, 4-nitrophenyl, 2-chloro-5-nitrophenyl, 2-hydroxyphenyl, 3-cyanophenyl, 4-cyanophenyl, 2-hydroxy-5-sulfamoylphenyl.

5. A process according to claim 1 characterized in that a compound of formula (I) is prepared wherein $R^c$ is hydroxy, methoxy, ethoxy, hydroxyamino or

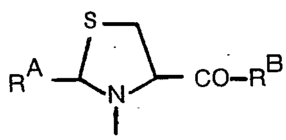 ;

6. A process according to claim 1 characterized in that a compound of formula (I) is prepared wherein $R^d$ is hydrogen, methyl, 2-phenylethyl or phenyl.

7. A process according to claim 1 characterized in that a compound of formula (I) is prepared wherein W is $—CH(CH_3)—CH_2—$, $—CH(CH_3)—(CH_2)_2—$, $—(CH_2)_{1-8}$, $—(CH_2)_{10}$ or $—(CH_2)_{12}$.

8. A process according to claim 1 characterized in that a compound of formula (I) is prepared wherein W is $—CH_2COCH(COCH_3)—$.

9. A process according to claim 1 characterized in that a compound of formula (I) is prepared wherein W is $—CH_2—O—CH_2—$, $—(CH_2)_2—O—(CH_2)_2—$, $—(CH_2)_2—S—(CH_2)_2—$ or $—(CH_2)_2—S—(CH_2)_2—S—(CH_2)_2—$.

10. A process according to claim 1 characterized in that a compound of formula (I) is prepared wherein W is

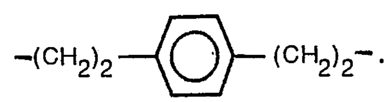

11. A process according to claim 1 characterized in that a compound of formula (I) is prepared wherein W is $—CH(CH_3)—S—CH(Ph)—$, $—CH_2—S—CH(CH_2CH_2Ph)—$, $—CH(CH_3)—S—CH(CH_2CH_2Ph)—$, $—CH(CH_2Ph)—S—CH_2—$, or $—CH(CH_2CH_2Ph)—S—CH_2—$.

12. A process according to claim 1 characterized in that a compound of formula (I) is prepared wherein W is

$—CH_2NHCH_2—$, 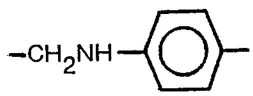 or $—CH_2NH—CH(CH_2CH_2Ph)—$.

13. A process according to claim 1 characterized in that (4R) - 3 - [8 - (ethoxycarbonyl) - octanoyl] - 2 - (3 - nitrophenyl) - 4 - thiazolidinecarboxylic acid is prepared.

14. A process according to claim 1 characterized in that (4R,4′R) - 3,3′ - (nonanedioyl)bis[2 - (3 - nitrophenyl) - 4 - thiazolidinecarboxylic acid methyl ester] is prepared.

15. A process according to claim 1 characterized in that (4R) - 3 - (11 - carboxyundecanoyl) - 2 - (3 - cyanophenyl) - 4 - thiazolidinecarboxylic acid is prepared.

16. A process according to claim 1 characterized in that (4R,4′R) - 3,3′ - (decanedioyl)bis[2 - (3 - cyanophenyl) - 4 - thiazolidinecarboxylic acid] is prepared.

17. A process according to claim 1 characterized in that (4R,4′R) - 3,3′ - (dodecanedioyl)bis[2 - (3 - cyanophenyl) - 4 - thiazolidinecarboxylic acid] is prepared.

18. A process according to claim 1 characterized in that (4R) - 3 - (8 - carboxyoctanoyl) - 2 - (3 - nitrophenyl) - 4 - thiazolidinecarboxylic acid is prepared.

19. A process according to claim 1 characterized in that (4R,4'R) - 3,3' - (nonanedioyl)bis[2 - (3 - nitrophenyl) - 4 - thiazolidinecarboxylic acid] is prepared.

20. A process according to claim 1 characterized in that (4R) - 3 - (7 - carboxyheptanoyl) - 2 - (2 - hydroxyphenyl) - 4 - thiazolidinecarboxylic acid is prepared.

21. A process according to claim 1 characterized in that (4R) - 3 - [[(1 - carboxy - 3 - phenylpropyl)-amino]acetyl] - 2 - (2 - hydroxyphenyl) - 4 - thiazolidinecarboxylic acid is prepared.

22. A process according to claim 1 characterized in that (4R) - 3 - [[(1 - carboxy - 3 - phenylpropyl)-thio]acetyl] - 2 - (2 - hydroxyphenyl) - 4 - thiazolidine-carboxylic acid is prepared.

23. A process according to claim 1 characterized in that (4R) - 3 - (4 - carboxybutanoyl) - 2 - (2 - hydroxyphenyl) - 4 - thiazolidinecarboxylic acid is prepared.

**Patentansprüche für die Vertragsstaaten: BE CH DE FR GB IT LI LU NL SE**

1. Eine Verbindung der Formel (I)

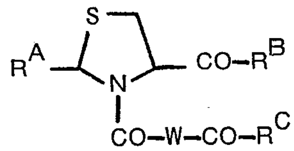

(I)

worin

$R^A$ für $R^a$ oder $R^b$ steht;

$R^B$ und $R^C$ jeweils für $R^c$ stehen;

W 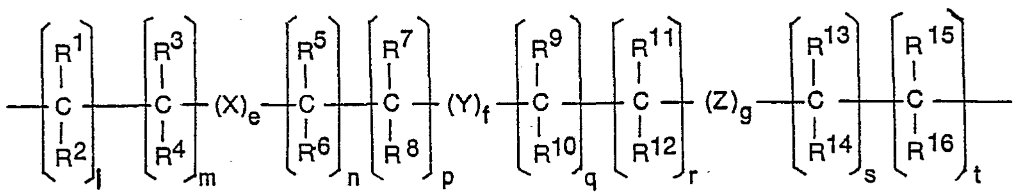

bedeutet, worin

X, Y und Z jeweils für

$-CH_2-$, 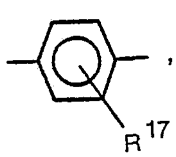 , 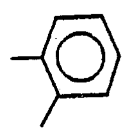 ,

$-O-$, $-CO-$, $-S-$, $-SO-$, $-SO_2-$, 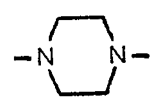 oder $-N(R^{18})-$ ;

stehen;

wobei, falls einer der Reste X, Y und Z für

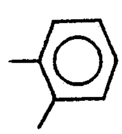 steht, $R^A$ 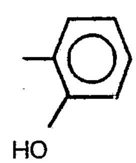 bedeutet,

$R^B$ und $R^C$ beide Hydroxy darstellen und W durch

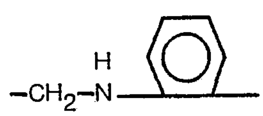 ;

dargestellt wird;

e, f und g jeweils 0 oder 1 bedeuten;

l, m, n, p, q, r, s und t jeweils 0, 1, 2 oder 3 sind, (l + m) aber nicht 0 ist;
$R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$ und $R^{18}$ jeweils $R^d$ sind;

falls W für 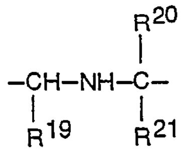 oder 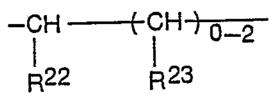 steht,

worin
$R^{19}$, $R^{20}$, $R^{21}$, $R^{22}$ und $R^{23}$ jeweils $R^d$ bedeuten, $R^A$, $R^b$ ist;
falls X für S steht und e 1 ist, (n + p) nicht 0 ist;
falls X S ist, e 1 ist und

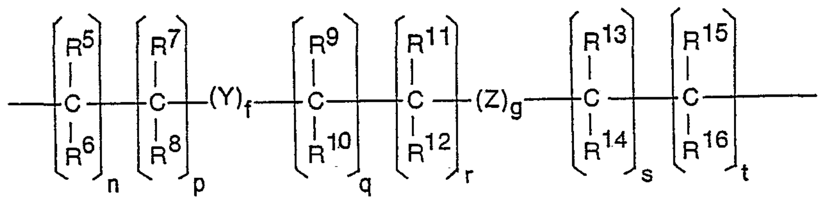

Niederalkylen bedeutet, $R^C$ nicht Hydroxy ist;
$R^a$ Wasserstoff oder $(C_1$—$C_4)$Alkyl bedeutet;
$R^b$ ausgewählt ist unter
(a) (i) Benzyl oder Phenylethyl und
(ii) Benzyl oder Phenylethyl, die durch wenigstens einen Substituenten, ausgewählt unter Methyl, Hydroxy, Methoxy, Halogen, Nitro und Cyano, substituiert sind, und
(b) Phenyl und durch wenigstens einen Substituenten, ausgewählt unter Methyl, Hydroxy, Methoxy, Benzyloxy, Acetyloxy, Halogen, Nitro, Cyano, Amino, Dimethylamino, Carboxy, Sulfamoyl, Difluormethoxy und Dipropylaminosulfonyl, substituiertem Phenyl;
$R^c$ ausgewählt ist unter
(a) Hydroxy, $(C_1$—$C_4)$Alkoxy, Amino, Hydroxyamino und Benzyloxy, und

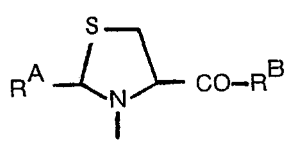 ; (b)

$R^d$ ausgewählt ist unter
(a) Wasserstoff, $(C_1$—$C_6)$Alkyl, Benzyl, Phenylethyl, Acetyl, Hydroxy, Carboxy und Amino, und
(b) Phenyl und durch wenigstens einen Substituenten, ausgewählt unter Methyl, Methoxy, Hydroxy, Carboxy, Amino, Halogen, Nitro und Cyano, substituiertem Phenyl, und deren Salze.

2. Eine Verbindung nach Anspruch 1 mit der folgenden Formel:

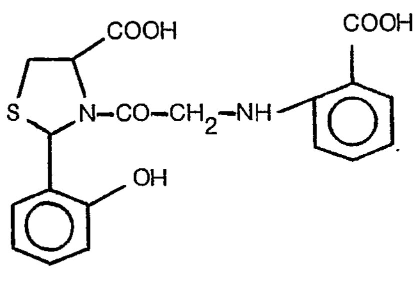

3. Eine Verbindung nach Anspruch 1, worin $R^a$ Wasserstoff bedeutet.

4. Eine Verbindung nach Anspruch 1, worin $R^b$ 2-Phenylethyl, Phenyl, 2-Fluorphenyl, 3-Fluorphenyl, 4-Fluorphenyl, 2-Nitrophenyl, 3-Nitrophenyl, 4-Nitrophenyl, 2-Chlor-5-nitrophenyl, 2-Hydroxyphenyl, 3-Cyanophenyl, 4-Cyanophenyl, 2-Hydroxy-5-sulfamoylphenyl bedeutet.

5. Eine Verbindung nach Anspruch 1, worin $R^c$ Hydroxy, Methoxy, Ethoxy, Hydroxyamino oder

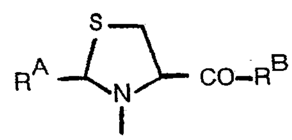 ;

bedeutet

6. Eine Verbindung nach Anspruch 1, worin $R^d$ Wasserstoff, Methyl, 2-Phenylethyl oder Phenyl bedeutet.

7. Eine Verbindung nach Anspruch 1, worin W für

$—CH(CH_3)—CH_2—$, $—CH(CH_3)—(CH_2)_2—$, $—(CH_2)_{7–8}$, $—(CH_2)_{10}$ oder $—(CH_2)_{12}$ steht.

8. Eine Verbindung nach Anspruch 1, worin W für $—CH_2COCH(COCH_3)—$ steht.

9. Eine Verbindung nach Anspruch 1, worin W für

$—CH_2—O—CH_2—$, $—(CH_2)_2—O—(CH_2)_2—$, $—(CH_2)_2—S—(CH_2)_2—$ oder $—(CH_2)_2—S—(CH_2)_2—S—(CH_2)_2—$ steht.

10. Eine Verbindung nach Anspruch 1, worin W für

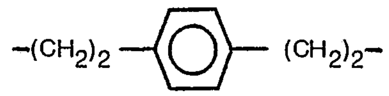 steht.

11. Eine Verbindung nach Anspruch 1, worin W für

$—CH(CH_3)—S—CH(Ph)—CH_2—S—CH(CH_2CH_2Ph)—$, $—CH(CH_3)—S—CH(CH_2CH_2Ph)—$, $—CH(CH_2Ph)—S—CH_2—$ oder $—CH(CH_2CH_2Ph)—S—CH_2—$ steht.

12. Eine Verbindung nach Anspruch 1, worin W für

$-CH_2NHCH_2-$, 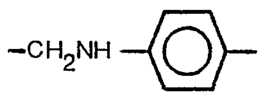 oder $-CH_2NH-CH(CH_2CH_2Ph)-$ steht.

13. Eine Verbindung nach Anspruch 1, welche (4R)-3-[8-Ethoxycarbonyl)-octanoyl]-2-(3-nitrophenyl)-4-thiazolidin-carbonsäure ist.

14. Eine Verbindung nach Anspruch 1, welche (4R,4'R)-3,3'-(Nonandioyl)bis[2-(3-nitrophenyl)-4-thiazolidincarbonsäuremethylester] ist.

15. Eine Verbindung nach Anspruch 1, welche (4)-3-(11-Carboxyundecanoyl)-2-(3-cyanophenyl)-4-thiazolidincarbonsäure ist.

16. Eine Verbindung nach Anspruch 1, welche (4R,4'R)-3,3'-(Decandioyl)bis[2-(3-cyanophenyl)-4-thiazolidincarbonsäure] ist.

17. Eine Verbindung nach Anspruch 1, welche (4R,4'R)-3,3'-(Dodecandioyl)bis[2-(3-cyanophenyl)-4-thiazolidincarbonsäure] ist.

18. Eine Verbindung nach Anspruch 1, welche (4R)-3-(8-Carboxyoctanoyl)-2-(3-nitrophenyl)-4-thiazolidincarbonsäure ist.

19. Eine Verbindung nach Anspruch 1, welche (4R,4'R)-3,3'-(Nonandioyl)bis[2-(3-nitrophenyl)-4-thiazolidincarbonsäure] ist.

20. Eine Verbindung nach Anspruch 1, welche (4R)-3-(7-Carboxyheptanoyl)-2-(2-hydroxyphenyl)-4-thiazolidincarbonsäure ist.

21. Eine Verbindung nach Anspruch 1, welche (4R)-3-[[(-1-Carboxy-3-phenylpropyl)amino]acetyl]-2-(2-hydroxyphenyl)-4-thiazolidincarbonsäure ist.

22. Eine Verbindung nach Anspruch 1, welche (4R)-3-[[(1-Carboxy-3-phenylpropyl)thio]acetyl]-2-(2-hydroxyphenyl)-4-thiazolidincarbonsäure ist.

23. Eine Verbindung nach Anspruch 1, welche (4R)-3-(4-Carboxy-butanoyl)-2-(2-hydroxyphenyl)-4-thiazolidincarbonsäure ist.

24. Ein Verfahren zur Herstellung einer Verbindung der Formel (I) gemäß Anspruch 1, und von deren Salzen, welches umfaßt

(i) Umsetzen einer Verbindung der Formel (II)

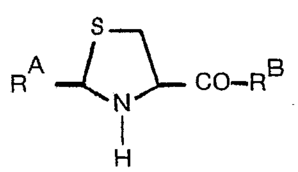 (II)

worin $R^A$ und $R^B$ einen geeigneten Schutz von etwaigen reaktionsfähigen Gruppen einschließen können, mit dem reaktionsfähigen Derivat einer Carbonsäure der Formel (III)

$$HOOC—W—CO—R^C \qquad (III)$$

worin $R^C$ und W einen geeigneten Schutz von etwaigen reaktionsfähigen Gruppen einschließen können, mit anschließender Entfernung von Schutzgruppen, falls erforderlich, um eine Verbindung der Formel (I) zu erhalten;

(ii) Umsetzen einer Verbindung der Formel (II) mit dem reaktionsfähigen Derivat einer Carbonsäure der Formel (IV)

$$HOOC—W^1—L \qquad (IV)$$

worin

$W^1$ für 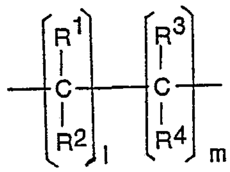 ,

steht und einen geeigneten Schutz von etwaigen reaktionsfähigen Gruppen einschließen kann, und L eine Leaving-Gruppe bedeutet, um eine Verbindung der Formel (V)

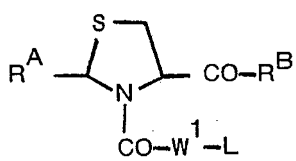

(V)

zu ergeben, und anschließende Umsetzung einer Verbindung der Formel (V) mit einer Verbindung der Formel (VI)

$$(H)(X)_eW^2—(Y)_f—W^3—(Z)_g—W^4\text{-}CO—R^C, \qquad (VI)$$

worin

$W^2$ für 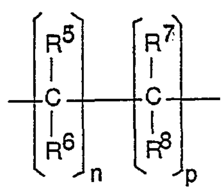 , $W^3$ für 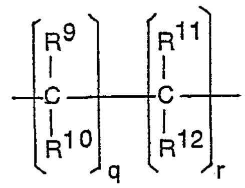 , $W^4$ für 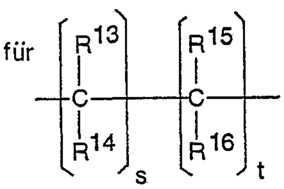

steht und $W^2$, $W^3$, $W^4$, X, Y, Z und $R^C$ einen geeigneten Schutz von etwaigen reaktionsfähigen Gruppen einschließen können, mit anschließender Abtrennung von Schutzgruppen, falls erforderlich, um eine Verbindung der Formel (I) zu ergeben;

(iii) Umsetzen einer Verbindung der Formel (II) mit dem reaktionsfähigen Derivat einer Carbonsäure der Formel (VII)

$$HOOC—W^1—(X)_e—W^2—L \qquad (VII)$$

und anschließend mit einer Verbindung der Formel (VIII)

$$(H)(Y)_f—W^3—(Z)_g—W^4—CO—R^C \qquad (VIII)$$

nach der gleichen Methode wie vorstehend unter (ii) angegeben, um eine Verbindung der Formel (I) zu erhalten;

(iv) Umsetzen einer Verbindung der Formel (II) mit dem reaktionsfähigen Derivat einer Carbonsäure der Formel (IX)

$$HOOC—W^1—(X)_e—W^2—(Y)_f—W^3—L \qquad (IX)$$

und anschließend mit einer Verbindung der Formel (X)

$$(H)(Z)_g—W^4—CO—R^C \qquad (X)$$

nach der gleichen Methode wie vorstehend unter (ii) angegeben, um eine Verbindung der Formel (I) zu erhalten;

(v) Umsetzen einer Verbindung der Formel (II) mit dem reaktionsfähigen Derivat einer Carbonsäure der Formel (XI)

$$HOOC—W^1—(X)_e(H) \qquad (XI)$$

und anschließend mit einer Verbindung der Formel (XII)

$$L—W^2—(Y)_f—W^3—(Z)_g—W^4—CO—R^C \qquad (XII)$$

nach der gleichen Methode wie vorstehend unter (ii) angegeben, um eine Verbindung der Formel (I) zu erhalten;

(vi) Umsetzen einer Verbindung der Formel (II) mit dem reaktionsfähigen Derivat einer Carbonsäure der Formel (XIII)

$$HOOC—W^1—(X)_e—W^2—(Y)_f(H) \qquad (XIII)$$

und anschließend mit einer Verbindung der Formel (XIV)

$$L—W^3—(Z)_g—W^4—CO—R^C \qquad (XIV)$$

nach der gleichen Methode, wie vorstehend unter (ii) angegeben, um eine Verbindung der Formel (I) zu erhalten, oder

(vii) Umsetzen einer Verbindung der Formel (II) mit dem reaktionsfähigen Derivat einer Carbonsäure der Formel (XV)

$$HOOC—W^1—(X)_e—W^2—(Y)_f—W^3—(Z)_g(H) \qquad (XV)$$

und anschließend mit einer Verbindung der Formel (XVI)

$$L—W^4—CO—R^C \qquad (XVI)$$

nach der gleichen Methode, wie vorstehend unter (ii) angegeben, um eine Verbindung der Formel (I) zu erhalten;
weiterhin Überführen von $R^B$, $R^C$, X, Y und Z in andere funktionelle Gruppen nach den allgemeinen Methoden, falls erwünscht, um eine gewünschte Verbindung der Formel (I) zu erhalten.

25. Eine pharmazeutische Zusammensetzung, enthaltend eine Verbindung der Formel (I) gemäß Anspruch 1 oder Salze hievon.

26. Eine Zusammensetzung gemäß Anspruch 25, enthaltend eine Verbindung der Formel (I) oder Salze hievon in einer Menge, die zur Verhinderung oder Erleichterung von mit Diabetes mellitus assoziierten Komplikationen, bestehend aus grauem Star, Neuropathie, Nephropathie und Retinopathie, ausreicht, und einen oder mehrere pharmazeutisch annehmbare Exzipienten.

27. Eine Verbindung nach Anspruch 1 bis 23 zur Anwendung in einem Therapie- oder Prophylaxeverfahren.

28. Verwendung einer Verbindung gemäß Anspruch 1 bis 23 in einem Verfahren zur Herstellung pharmazeutischer Zusammensetzungen.

**Patentansprüche für den Vertragsstaat: AT**

1. Ein Verfahren zur Herstellung einer Verbindung der Formel (I)

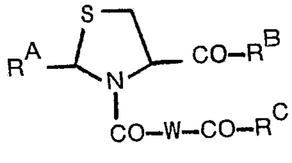

(I)

worin

$R^A$ für $R^a$ oder $R^b$ steht;

$R^B$ und $R^C$ jeweils für $R^c$ stehen;

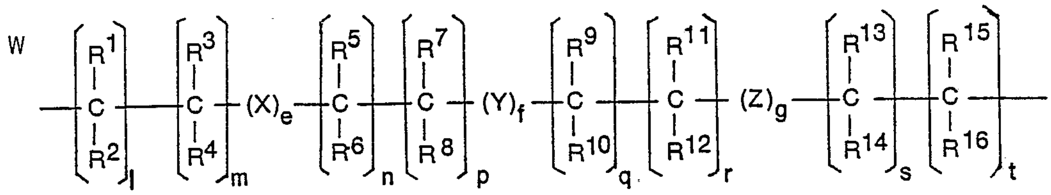

bedeutet, worin

X, Y und Z jeweils für

$-CH_2-$, 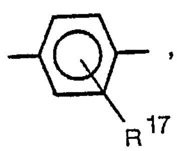 , 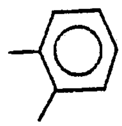 ,

$-O-$, $-CO-$, $-S-$, $-SO-$, $-SO_2-$, 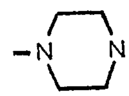 oder 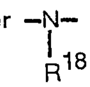 ;

stehen;
wobei, falls einer der Reste X, Y und Z für

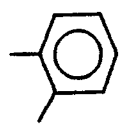 steht, $R^A$ 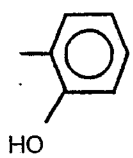 bedeutet,

$R^B$ und $R^C$ beide Hydroxy darstellen und W durch

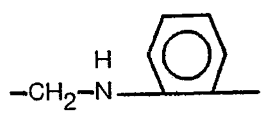 ;

dargestellt wird;

e, f und g jeweils 0 oder 1 bedeuten;

l, m, n, p, q, r, s und t jeweils 0, 1, 2 oder 3 sind, (l + m) aber nicht 0 ist;

$R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$ und $R^{18}$ jeweils $R^d$ sind;

falls

W für 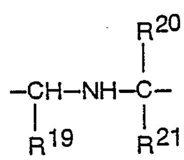 oder 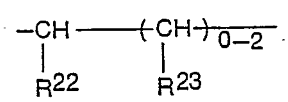 steht,

worin

$R^{19}$, $R^{20}$, $R^{21}$, $R^{22}$ und $R^{23}$ jeweils $R^d$ bedeuten, $R^A$, $R^b$ ist;

falls X für S steht und e 1 ist, (n + p) nicht 0 ist;

falls X S ist, e 1 ist und

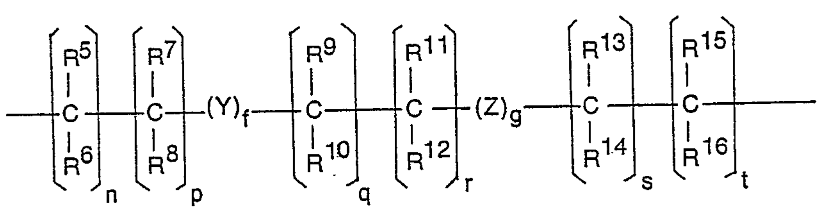

Niederalkylen bedeutet, $R^C$ nicht Hydroxy ist;

$R^a$ Wasserstoff oder $(C_1—C_4)$Alkyl bedeutet;

$R^b$ ausgewählt ist unter
(a) (i) Benzyl oder Phenylethyl und
(ii) Benzyl oder Phenylethyl, die durch wenigstens einen Substituenten, ausgewählt unter Methyl, Hydroxy, Methoxy, Halogen, Nitro und Cyano, substituiert sind, und
(b) Phenyl und durch wenigstens einen Substituenten, ausgewählt unter Methyl, Hydroxy, Methoxy, Benzyloxy, Acetyloxy, Halogen, Nitro, Cyano, Amino, Dimethylamino, Carboxy, Sulfamoyl, Difluormethoxy und Dipropylaminosulfonyl, substituiertem Phenyl;
$R^c$ ausgewählt ist unter
(a) Hydroxy, $(C_1—C_4)$Alkoxy, Amino, Hydroxyamino und Benzyloxy, und

(b)

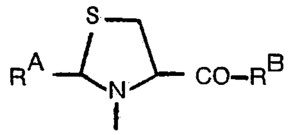 ;

$R^d$ ausgewählt ist unter
(a) Wasserstoff, $(C_1—C_6)$Alkyl, Benzyl, Phenylethyl, Acetyl, Hydroxy, Carboxy und Amino, und
(b) Phenyl und durch wenigstens einen Substituenten, ausgewählt unter Methyl, Methoxy, Hydroxy, Carboxy, Amino, Halogen, Nitro und Cyano, substituiertem Phenyl, und von deren Salzen, welches umfaßt
(i) Umsetzen einer Verbindung der Formel (II)

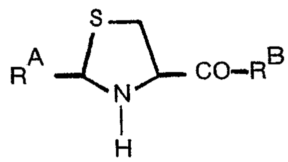 (II)

worin $R^A$ und $R^B$ einen geeigneten Schutz von etwaigen reaktionsfähigen Gruppen einschließen können, mit dem reaktionsfähigen Derivat einer Carbonsäure der Formel (III)

$$HOOC—W—CO—R^C \qquad (III)$$

worin $R^C$ und W einen geeigneten Schutz von etwaigen reaktionsfähigen Gruppen einschließen können, mit anschließender Entfernung von Schutzgruppen, falls erforderlich, um eine Verbindung der Formel (I) zu erhalten;

(ii) Umsetzen einer Verbindung der Formel (II) mit dem reaktionsfähigen Derivat einer Carbonsäure der Formel (IV)

$$HOOC—W^1—L \qquad (IV)$$

worin

$W^1$ fur 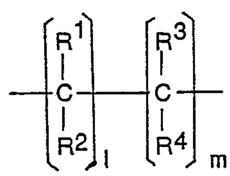 ,

steht und einen geeigneten Schutz von etwaigen reaktionsfähigen Gruppen einschließen kann, und L eine Leaving-Gruppe bedeutet, um eine Verbindung der Formel (V)

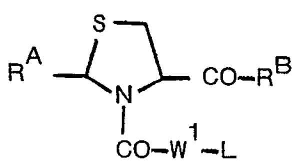 (V)

zu ergeben, und anschließende Umsetzung einer Verbindung der Formel (V) mit einer Verbindung der Formel (VI)

$$(H)(X)_eW^2—(Y)_f—W^3—(Z)_g—W^4—CO—R^C, \qquad (VI)$$

worin

$W^2$ für 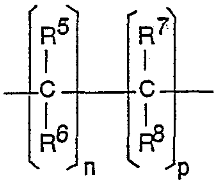, $W^3$ für 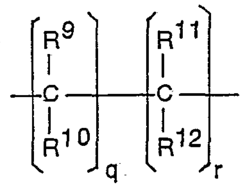, $W^4$ für 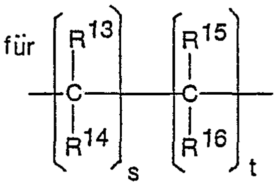.

steht und $W^2$, $W^3$, $W^4$, X, Y, Z und $R^C$ einen geeigneten Schutz von etwaigen reaktionsfähigen Gruppen einschließen können, mit anschließender Abtrennung von Schutzgruppen, falls erforderlich, um eine Verbindung der Formel (I) zu ergeben;

(iii) Umsetzen einer Verbindung der Formel (II) mit dem reaktionsfähigen Derivat einer Carbonsäure der Formel (VII)

$$HOOC—W^1—(X)_e—W^2—L \qquad (VII)$$

und anschließend mit einer Verbindung der Formel (VIII)

$$(H)(Y)_f—W^3—(Z)_g—W^4—CO—R^C \qquad (VIII)$$

nach der gleichen Methode wie vorstehend unter (ii) angegeben, um eine Verbindung der Formel (I) zu erhalten;

(iv) Umsetzen einer Verbindung der Formel (II) mit dem reaktionsfähigen Derivat einer Carbonsäure der Formel (IX)

$$HOOC—W^1—(X)_e—W^2—(Y)_f—W^3—L \qquad (IX)$$

und anschließend mit einer Verbindung der Formel (X)

$$(H)(Z)_g—W^4—CO—R^C \qquad (X)$$

nach der gleichen Methode wie vorstehend unter (ii) angegeben, um eine Verbindung der Formel (I) zu erhalten;

(v) Umsetzen einer Verbindung der Formel (II) mit dem reaktionsfähigen Derivat einer Carbonsäure der Formel (XI)

$$HOOC—W^1—(X)_e(H) \qquad (XI)$$

und anschließend mit einer Verbindung der Formel (XII)

$$L—W^2—(Y)_f—W^3—(Z)_g—W^4—CO—R^C \qquad (XII)$$

nach der gleichen Methode wie vorstehend unter (ii) angegeben, um eine Verbindung der Formel (I) zu erhalten;

(vi) Umsetzen einer Verbindung der Formel (II) mit dem reaktionsfähigen Derivat einer Carbonsäure der Formel (XIII)

$$HOOC—W^1—(X)_e—W^2—(Y)_f(H) \qquad (XIII)$$

und anschließend mit einer Verbindung der Formel (XIV)

$$L—W^3—(Z)_g—W^4—CO—R^C \qquad (XIV)$$

nach der gleichen Methode, wie vorstehend unter (ii) angegeben, um eine Verbindung der Formel (I) zu erhalten, oder

(vii) Umsetzen einer Verbindung der Formel (II) mit dem reaktionsfähigen Derivat einer Carbonsäure der Formel (XV)

$$HOOC—W^1—(X)_e—W^2—(Y)_f—W^3—(Z)_g(H) \qquad (XV)$$

und anschließend mit einer Verbindung der Formel (XVI)

$$L—W^4—CO—R^C \qquad (XVI)$$

nach der gleichen Methode, wie vorstehend unter (ii) angegeben, um eine Verbindung der Formel (I) zu erhalten;
weiterhin Überführen von $R^B$, $R^C$, X, Y und Z in andere funktionelle Gruppen nach den allgemeinen Methoden, falls erwünscht, um eine gewünschte Verbindung der Formel (I) zu erhalten.

2. Ein Verfahren nach Anspruch 1, dadurch gekennzeichnet, daß eine Verbindung mit der folgenden Formel:

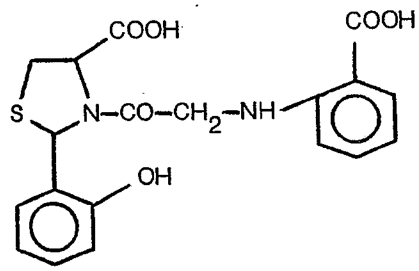

hergestellt wird.

3. Ein Verfahren nach Anspruch 1, dadurch gekennzeichnet, daß eine Verbindung der Formel (I) hergestellt wird, worin $R^a$ Wasserstoff bedeutet.

4. Ein Verfahren nach Anspruch 1, dadurch gekennzeichnet, daß eine Verbindung der Formel (I) hergestellt wird, worin $R^b$ 2-Phenylethyl, Phenyl, 2-Fluorphenyl, 3-Fluorphenyl, 4-Fluorphenyl, 2-Nitrophenyl, 3-Nitrophenyl, 4-Nitrophenyl, 2-Chlor-5-nitrophenyl, 2-Hydroxyphenyl, 3-Cyanophenyl, 4-Cyanophenyl, 2-Hydroxy-5-sulfamoylphenyl bedeutet.

5. Ein Verfahren nach Anspruch 1, dadurch gekennzeichnet, daß eine Verbindung der Formel (I) hergestellt wird, worin $R^c$ Hydroxy, Methoxy, Ethoxy, Hydroxyamino oder

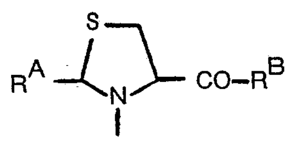 ;

bedeutet

6. Ein Verfahren nach Anspruch 1, dadurch gekennzeichnet, daß eine Verbindung der Formel (I) hergestellt wird, worin $R^d$ Wasserstoff, Methyl, 2-Phenylethyl oder Phenyl bedeutet.

7. Ein Verfahren nach Anspruch 1, dadurch gekennzeichnet, daß eine Verbindung der Formel (I) hergestellt wird, worin W für

$-CH(CH_3)-CH_2-$, $-CH(CH_3)-(CH_2)_2-$, $-(CH_2)_{1-8}-$, $-(CH_2)_{10}-$
oder $-(CH_2)_{12}-$ steht.

8. Ein Verfahren nach Anspruch 1, dadurch gekennzeichnet, daß eine Verbindung der Formel (I) hergestellt wird, worin W für $-CH_2COCH(COCH_3)-$ steht.

9. Ein Verfahren nach Anspruch 1, dadurch gekennzeichnet, daß eine Verbindung der Formel (I) hergestellt wird, worin W für

$-CH_2-O-CH_2-$, $-(CH_2)_2-O-(CH_2)_2-$, $-(CH_2)_2-S-(CH_2)_2-$ oder
$-(CH_2)_2-S-(CH_2)_2-S-(CH_2)_2-$ steht.

10. Ein Verfahren nach Anspruch 1, dadurch gekennzeichnet, daß eine Verbindung der Formel (I) hergestellt wird, worin W für

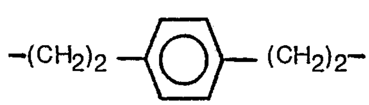 steht.

11. Ein Verfahren nach Anspruch 1, dadurch gekennzeichnet, daß eine Verbindung der Formel (I) hergestellt wird, worin W für

$-CH(CH_3)-S-CH(Ph)-$, $CH_2-S-CH(CH_2CH_2Ph)-$, $-CH(CH_3)-S-CH(CH_2CH_2Ph)-$,
$-CH(CH_2Ph)-S-CH_2-$ oder $-CH(CH_2CH_2Ph)-S-CH_2-$ steht.

12. Ein Verfahren nach Anspruch 1, dadurch gekennzeichnet, daß eine Verbindung der Formel (I) hergestellt wird, worin W für

$-CH_2NHCH_2-$, 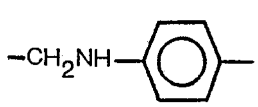 oder $-CH_2NH-CH(CH_2CH_2Ph)-$ steht.

13. Ein Verfahren nach Anspruch 1, dadurch gekennzeichnet, daß (4R)-3-[8-Ethoxycarbonyl)-octanoyl]-2-(3-nitrophenyl)-4-thiazolidin-carbonsäure hergestellt wird.

14. Ein Verfahren nach Anspruch 1, dadurch gekennzeichnet, daß (4R,4'R)-3,3'-(Nonandioyl)bis[2-(3-nitrophenyl)-4-thiazoldincarbonsäuremethylester] hergestellt wird.

15. Ein Verfahren nach Anspruch 1, dadurch gekennzeichnet, daß (4R)-3-(11-Carboxyundecanoyl)-2-(3-cyanophenyl)-4-thiazolidincarbonsäure hergestellt wird.

16. Ein Verfahren nach Anspruch 1, dadurch gekennzeichnet, daß (4R,4'R)-3,3'-(Decandioyl)bis[2-(3-cyanophenyl)-4-thiazolidincarbonsäure hergestellt wird.

17. Ein Verfahren nach Anspruch 1, dadurch gekennzeichnet, daß (4R,4'R)-3,3'-(Dodecandioyl)bis[2-(3-cyanophenyl)-4-thiazolidincarbonsäure] hergestellt wird.

18. Ein Verfahren nach Anspruch 1, dadurch gekennzeichnet, daß (4R)-3-(8-Carboxyoctanoyl)-2-(3-nitrophenyl)-4-thiazolidincarbonsäure hergestellt wird.

19. Ein Verfahren nach Anspruch 1, dadurch gekennzeichnet, daß (4R,4'R)-3,3'-(Nonandioyl)bis[2-(3-nitrophenyl)-4-thiazolidincarbonsäure]hergestellt wird.

20. Ein Verfahren nach Anspruch 1, dadurch gekennzeichnet, daß(4R)-3-(7-Carboxyheptanoyl)-2-(2-hydroxyphenyl)-4-thiazolidincarbonsäure hergestellt wird.

21. Ein Verfahren nach Anspruch 1, dadurch gekennzeichnet, daß(4R)-3-[[(-1-Carboxy-3-phenylpropyl)amino]acetyl]-2-(2-hydroxyphenyl)-4-thiazolidincarbonsäure.

22. Ein Verfahren nach Anspruch 1, dadurch gekennzeichnet, daß (4R)-3-[[(1-Carboxy-3-phenylpropyl)thio]acetyl]-2-(2-hydroxyphenyl)-4-thiazolidincarbonsäure hergestellt wird.

23. Ein Verfahren nach Anspruch 1, dadurch gekennzeichnet, daß (4R)-3-(4-Carboxybutanoyl)-2-(2-hydroxyphenyl)-4-thiazolidincarbonsäure hergestellt wird.

**Revendications pour les Etats contractants: BE CH DE FR GB IT LI NL SE**

1. Un composé de formule [I]:

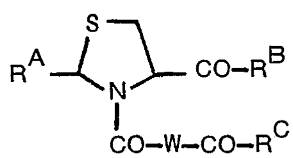

(I)

dans laquelle:

$R^A$ est $R^a$ ou $R^b$;

$R^B$ et $R^C$ sont chacun $R^c$;

W est

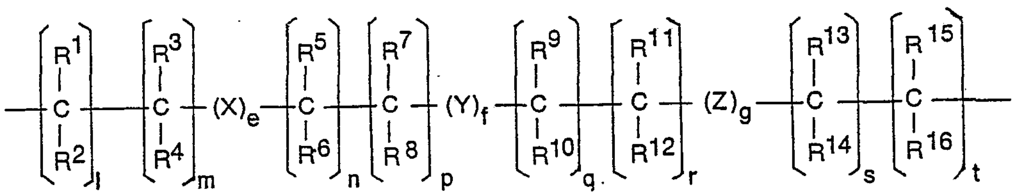

où

X, Y et Z sont chacun

$-CH_2-$, 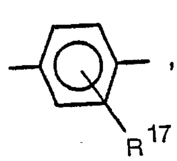, 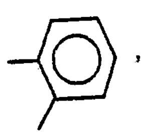,

$-O-$, $-CO-$, $-S-$, $-SO-$, $-SO_2-$, 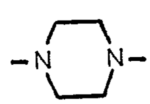 ou 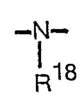 ;

si bien que lorsqu'un quelconque de X, Y et Z est

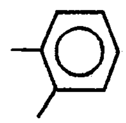 $R^A$ est 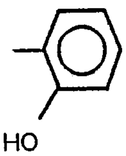 ,

$R^B$ et $R^C$ sont tous deux un hydroxy et W est représenté par

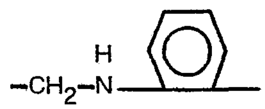 ;

e, f et g sont chacun 0 ou 1;
l, m, n, p, q, r, s et t sont chacun 0, 1, 2 ou 3, mais (l + m) n'est pas 0;
$R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$ et $R^{18}$ sont chacun $R^d$; lorsque

W est 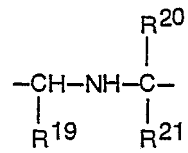 ou 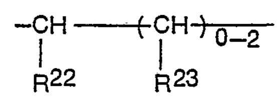

où $R^{19}$, $R^{20}$, $R^{21}$, $R^{22}$ et $R^{23}$ sont chacun $R^d$, $R^A$ est $R^b$ lorsque X est S et e est 1, (n + p) n'est pas 0; lorsque X est S, e est 1 et

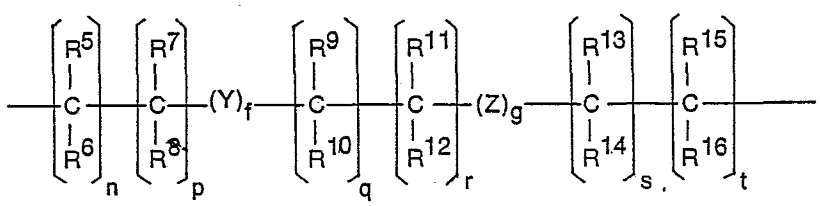

est un alcoylène inférieur, $R^C$ n'est pas un hydroxy;
$R^a$ est un hydrogène ou un alcoyle ($C_1$—$C_4$);
$R^b$ est choisi parmi
(a) (i) un benzyle ou un phényléthyle et
(ii) un benzyle ou un phényléthyle substitués par au moins un substituant choisi parmi méthyle, hydroxy, méthoxy, halogène, nitro et cyano, et
(b) un phényle et un phényle substitué par au moins un substituant choisi parmi méthyle, hydroxy, méthoxy, benzyloxy, acétyloxy, halogène, nitro, cyano, amino, diméthylamino, carboxy, sulfamoyle, difluorométhoxy et dipropylaminosulfonyle;
$R^c$ est choisi parmi
(a) un hydroxy, un alcoxy($C_1$—$C_4$), un amino, un hydroxyamino et un benzyloxy, et

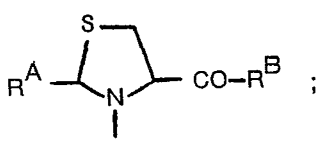 ; (b)

$R^d$ est choisi parmi
(a) un hydrogène, un alcoyle($C_1$—$C_6$), un benzyle, un phényléthyle, un acétyle, un hydroxy, un carboxy et un amino, et
(b) un phényle et un phényle substitué par au moins un substituant choisi parmi méthyle, méthoxy, hydroxy, carboxy, amino, halogène, nitro et cyano, et leurs sels.

2. Un composé de la revendication 1 ayant la formule suivante:

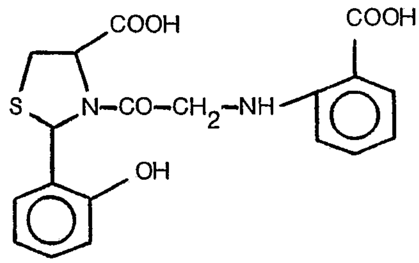

3. Un composé de la revendication 1 dans laquelle $R^a$ est un hydrogène.

4. Un composé de la revendication 1 dans laquelle $R^b$ est un 2-phényléthyle, un phényle, un 2-fluorophényle, un 3-fluorophényle, un 4-fluorophényle, un 2-nitrophényle, un 3-nitrophényle, un 4-nitrophényle, un 2-chloro-5-nitrophényle, un 2-hydroxyphényle, un 3-cyanophényle, un 4-cyanophényle, un 2-hydroxy-5-sulfamoylphényle.

5. Un composé de la revendication 1 dans laquelle $R^c$ est un hydroxy, un méthoxy, un éthoxy, un hydroxyamino ou

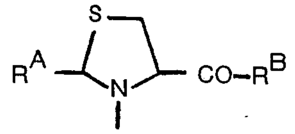 ;

6. Un composé de la revendication 1 dans laquelle $R^d$ est un hydrogène, un méthyle, un 2-phényléthyle ou un phényle.

7. Un composé de la revendication 1 dans laquelle W est $-CH(CH_3)-CH_2-$, $-CH(CH_3)-(CH_2)_2-$, $-(CH_2)_{1-8}$, $-(CH_2)_{10}-$ ou $-(CH_2)_{12}-$.

8. Un composé de la revendication 1 dans laquelle W est $-CH_2COCH(COCH_3)-$.

9. Un composé de la revendication 1 dans laquelle W est $-CH_2-O-CH_2-$, $-(CH_2)_2-O-(CH_2)_2-$, $-(CH_2)_2-S-(CH_2)_2-$ ou $-(CH_2)_2-S-(CH_2)_2-S-(CH_2)_2-$.

10. Un composé de la revendication 1 dans laquelle W est

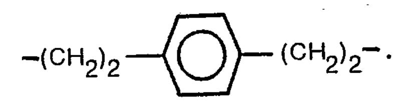 .

11. Un composé de la revendication 1 dans laquelle W est $-CH(CH_3)-S-CH(Ph)-$, $-CH_2-S-CH(CH_2CH_2Ph)-$, $-CH(CH_3)-S-CH(CH_2CH_2Ph)-$, $-CH(CH_2Ph)-S-CH_2-$, ou $-CH(CH_2CH_2Ph)-S-CH_2-$.

12. Un composé de la revendication 1 dans laquelle W est

$-CH_2NHCH_2-$, 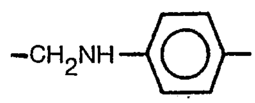 or $-CH_2NH-CH(CH_2CH_2Ph)-$.

13. Un composé de la revendication 1 qui est l'acide(4R)-3-[8-(éthoxycarbonyl)-octanoyl]-2-(3-nitrophényl)-4-thiazolidinecarboxylique.

14. Un composé de la revendication 1 qui est le (4R,4'R)-3,3'-(nonanedioyl)bis[2-(3-nitrophényl)-4-thiazolidinecarboxylate de méthyle].

15. Un composé de la revendication 1 qui est l'acide (4R)-3-(11-carboxyundécanoyl)-2-(3-cyanophényl)-4-thiazolidinecarboxylique.

16. Un composé de la revendication 1 qui est l'acide(4R,4'R)-3,3'-(décanedioyl)bis[2-(3-cyanophényl)-4-thiazolidinecarboxylique].

17. Un composé de la revendication 1 qui est l'acide(4R,4'R)-3,3'-dodécanedioyl)bis[2-(3-cyanophényl)-4-thiazolidinecarboxylique].

18. Un composé de la revendication 1 qui est l'acide(4R)-3-(8-carboxyoctanoyl)-2-(3-nitrophényl)-4-thiazolidinecarboxylique.

19. Un composé de la revendication 1 qui est l'acide(4R,4'R)-3,3'-(nonanedioyl)bus[2-(3-nitrophényl)-4-thiazolidinecarboxylique].

20. Un composé de la revendication 1 qui est l'acide(4R)-3-(7-carboxyheptanoyl)-2-(2-hyroxyphényl)-4-thiazolidinecarboxylique.

21. Un composé de la revendication 1 qui est l'acide(4R)-3-[[(1-carboxy-3-phénylpropyl)amino]acétyl]-2-(2-hydroxyphényl)-4-thiazolidinecarboxylique.

22. Un composé de la revendication 1 qui est l'acide(4R)-3-[[(1-carboxy-3-phénylpropyl)thio]acétyl]-2-(2-hydroxyphényl)-4-thiazolidinecarboxylique.

23. Un composé de la revendication 1 qui est l'acide (4R)-3-(4-carboxybutanoyl)-2-(2-hydroxyphényl)-4-thiazolidinecarboxylique.

24. Un procédé pour la préparation d'un composé de formule [I] selon la revendication 1 du brevet et ses sels, qui comprend

(i) la réaction d'un composé de formule [II]

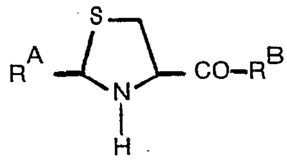

(II)

dans laquelle $R^A$ et $R^B$ peuvent comprendre une protection appropriée de tout groupe réactif avec le dérivé réactif d'un acide carboxylique de formule [III]

$$HOOC—W—CO—R^C \qquad [III]$$

dans laquelle $R^C$ et W peuvent comprendre une protection appropriée de tout groupe réactif, suivie de l'élimination des groupes protecteurs, au besoin, pour fournir un composé de formule [I];

(ii) la réaction d'un composé de formule [II] avec le dérivé réactif d'acide carboxylique de formule [IV]

$$HOOC—W^1—L \qquad [IV]$$

dans laquelle

$W^1$ est 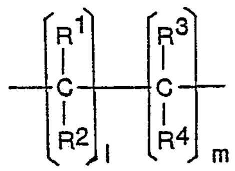 ,

et peut comprendre une protection appropriée de tout groupe réactif et L est un groupe labile pour fournir un composé de formule [V]

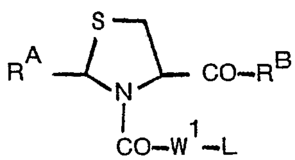

(V)

puis la réaction d'un composé de formule [V] avec un composé de formule [VI]

$$(H)(X)_eW^2—(Y)_f—W^3—(Z)_g—W^4—CO—R^C \qquad [VI]$$

dans laquelle

$W^2$ est 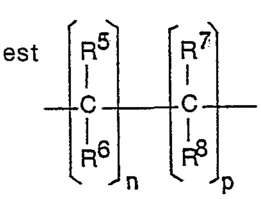 , $W^3$ est 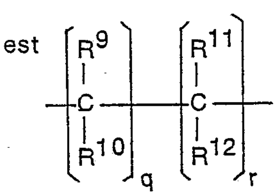 , $W^4$ est 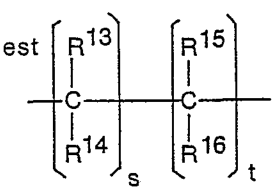

et $W^2$, $W^3$, $W^4$, X, Y, Z et $R^C$ peuvent comprendre une protection appropriée de tout groupe réactif, suivie de l'élimination des groupes protecteurs, au besoin, pour fournir un composé de formule [I];

(iii) la réaction d'un composé de formule [II] avec le dérivé réactif d'acide carboxylique de formule [VII]

$$HOOC—W^1—(X)_e—W^2—L \qquad [VII]$$

puis avec un composé de formule [VIII]

$$(H)(Y)—W^3—(Z)_g—W^4—CO—R^C \qquad [VIII]$$

selon le même procédé qu'en (ii) ci-dessus pour fournir un composé de formule [I];

(iv) la réaction d'un composé de formule [II] avec le dérivé réactif d'acide carboxylique de formule [IX]

$$HOOC—W^1—(X)_e—W^2—(Y)_f—W^3—L \qquad [IX]$$

puis avec un composé de formule [X]

$$(H)(Z)_g—W^4—CO—R^C \qquad [X]$$

selon le même procédé qu'en (ii) ci-dessus pour fournir un composé de formule [I];

(v) la réaction d'un composé de formule [II] avec le dérivé réactif d'acide carboxylique de formule [XI]

$$HOOC—W^1—(X)_e(H) \qquad [XI]$$

puis avec un composé de formule [XII]

$$L—W^2—(Y)_f—W^3—(Z)_g—W^4—CO—R^C \qquad [XII]$$

selon le même procédé qu'en (ii) ci-dessus pour fournir un composé de formule [I];

(vi) la réaction d'un composé de formule [II] avec le dérivé réactif d'acide carboxylique de formule [XIII]

$$HOOC—W^1—(X)_e—W^2—(Y)_f(H) \qquad [XIII]$$

puis avec un composé de formule [XIV]

$$L—W^3—(Z)_g—W^4—CO—R^C \qquad [XIV]$$

selon le même procédé qu'en (ii) ci-dessus pour fournir un composé de formule [I] ou

(vii) la réaction d'un composé de formule [II] avec le dérivé réactif d'acide carboxylique de formule [XV]

$$HOOC—W^1—(X)_e—W^2—(Y)_f—W^3—(Z)_g(H) \qquad [XV]$$

puis avec un composé de formule [XVI]

$$L—W^4—CO—R^C \qquad [XVI]$$

selon le même procédé qu'en (ii) ci-dessus pour fournir un composé de formule [I]; et de plus la conversion de $R^B$, $R^C$, X, Y et Z en d'autres groupes fonctionnels selon des procédés généraux, au besoin, pour obtenir un composé désiré de formule [I].

25. Une composition pharmaceutique comprenant un composé de formule [I] selon la revendication 1 du brevet ou ses sels.

26. Une composition selon la revendication 25 du brevet comprenant un composé de formule [I] ou ses sels en une quantité suffisante pour éviter ou réduire les complications associées au diabète sucré consistant en la cataracte, une neuropathie, une néphropathie et une rétinopathie, et un ou plusieurs excipients convenant en pharmacie.

27. Un composé selon les revendications 1 à 23 pour l'emploi dans un procédé de thérapeutique ou de prophylaxie.

28. L'emploi d'un composé selon les revendications 1 à 23 dans un procédé de production de compositions pharmaceutiques.

**Revendications pour l'Etat contractant: AT**

1. Un procédé pour préparer un composé de formule [I]

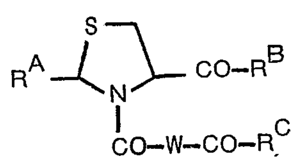

(I)

dans laquelle:

$R^A$ est $R^a$ ou $R^b$;

$R^B$ et $R^C$ sont chacun $R^c$;

W est 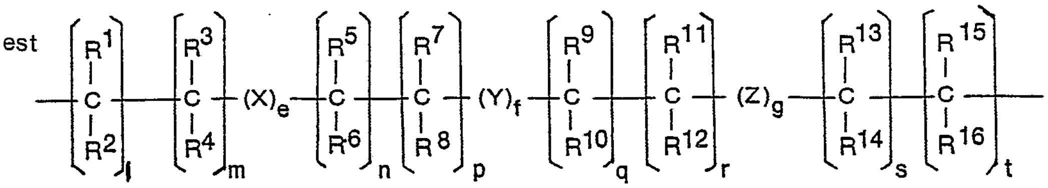

où

X, Y et Z sont chacun

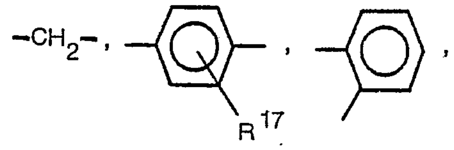

—O—, —CO—, —S—, —SO—, —$SO_2$—, 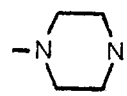 ou 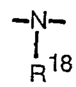 ;

si bien que lorsqu'un quelconque de X, Y et Z est

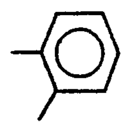 $R^A$ est 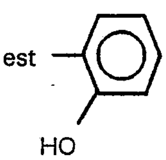 ,

$R^B$ et $R^C$ sont tous deux un hydroxy et W est représenté par

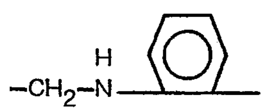 ;

e, f et g sont chacun 0 ou 1;

l, m, n, p, q, r, s et t sont chacun 0, 1, 2 ou 3, mais (l + m) n'est pas 0;

$R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$ et $R^{18}$ sont chacun $R^d$; lorsque

W est 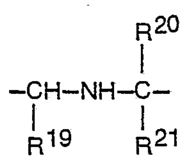 ou 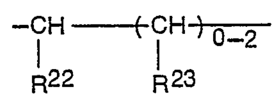

où $R^{19}$, $R^{20}$, $R^{21}$, $R^{22}$ et $R^{23}$ sont chacun $R^d$, $R^A$ est Rb lorsque X est S et e est 1, (n + ) n'est pas 0; lorsque X est S, e est 1 et

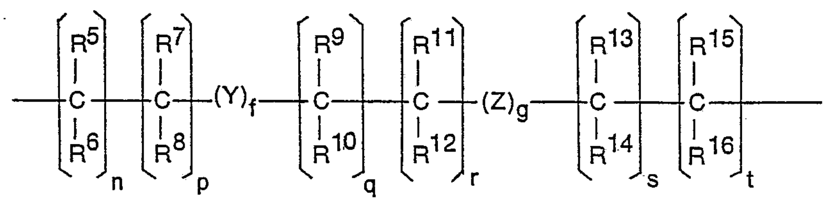

est un alcoylène inférieur, $R^C$ n'est pas un hydroxy;

$R^a$ est un hydrogène ou un alcoyle ($C_1$—$C_4$);

$R^b$ est choisi parmi

(a) (i) un benzyle ou un phényléthyle et
(ii) un benzyle ou un phényléthyle substitués par au moins un substituant choisi parmi méthyle, hydroxy, méthoxy, halogène, nitro et cyano, et
(b) un phényle et un phényle substitué par au moins un substituant choisi parmi méthyle, hydroxy, méthoxy, benzyloxy, acétyloxy, halogène, nitro, cyano, amino, diméthylamino, carboxy, sulfamoyle, difluorométhoxy et dipropylaminosulfonyle;

$R^c$est choisi parmi
(a) un hydroxy, un alcoxy($C_1$—$C_4$), un amino, un hydroxyamino et un benzyloxy, et

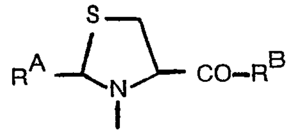 ; (b)

$R^d$ est choisi parmi
(a) un hydrogène, un alcoyle($C_1$—$C_6$), un benzyle, un phényléthyle, un acétyle, un hydroxy, un carboxy et un amino, et
(b) un phényle et un phényle substitué par au moins un substituant choisi parmi méthyle, méthoxy, hydroxy, carboxy, amino, halogène, nitro et cyano, et leurs sels qui comprend
(i) la réaction d'un composé de formule [II]

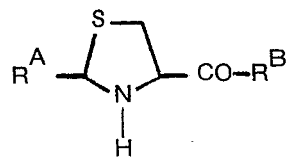 (II)

dans laquelle $R^A$ et $R^B$ peuvent comprendre une protection appropriée de tout groupe réactif, avec le dériyé réactif d'un acide carboxylique de formule [III]

HOOC—W—CO—$R^C$ [III]

dans laquelle $R^C$ et W peuvent comprendre une protection appropriée de tout groupe réactif, suivie de l'élimination des groupes protecteurs, au besoin, pour fournir un composé de formule [I];
(ii) la réaction d'un composé de formule [II] avec le dérivé réactif d'acide carboxylique de formule [IV]

HOOC—$W^1$—L [IV]

dans laquelle

$W^1$ est 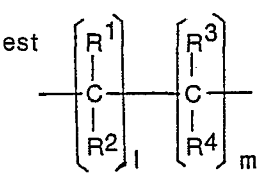 ,

et peut comprendre une protection appropriée de tout groupe réactif et L est un groupe labile, pour fournir un composé de formule [V]

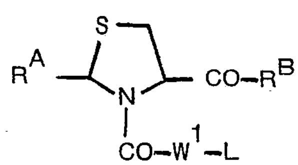 (V)

puis la réaction d'un composé de formule [V] avec un composé de formule [VI]

$(H)(X)_eW^2—(Y)_f—W^3—(Z)_g—W^4—CO—R^C$ [VI]

dans laquelle

$W^2$ est 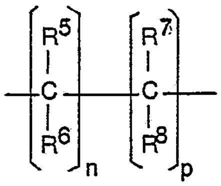 , $W^3$ est 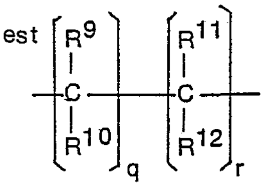 , $W^4$ est 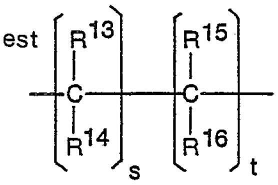 .

et $W^2$, $W^3$, $W^4$, X, Y, Z et $R^C$ peuvent comprendre une protection appropriée de tout groupe réactif, suivie de l'élimination des groupes protecteurs, au besoin, pour fournir un composé de formule [I];

(iii) la réaction d'un composé de formule [II] avec le dérivé réactif d'acide carboxylique de formule [VII]

$$HOOC—W^1—(X)_e—W^2—L \qquad [VII]$$

puis avec un composé de formule [VIII]

$$(H)(Y)_f—W^3—(Z)_g—W^4—CO—R^C \qquad [VIII]$$

selon le même procédé qu'en (ii) ci-dessus pour fournir un composé de formule [I];

(iv) la réaction d'un composé de formule [II] avec le dérivé réactif d'acide carboxylique de formule [IX]

$$HOOC—W^1—(X)_e—W^2—(Y)_f—W^3—L \qquad [IX]$$

puis avec un composé de formule [X]

$$(H)(Z)_g—W^4—CO—R^C \qquad [X]$$

selon le même procédé qu'en (ii) ci-dessus pour fournir un composé de formule [I];

(v) la réaction d'un composé de formule [II] avec le dérivé réactif d'acide carboxylique de formule [XI]

$$HOOC—W^1—(X)_e(H) \qquad [XI]$$

puis avec un composé de formule [XII]

$$L—W^2—(Y)_f—W^3—(Z)_g—W^4—CO—R^C \qquad [XII]$$

selon le même procédé qu'en (ii) ci-dessus pour fournir un composé de formule [I];

(vi) la réaction d'un composé de formule [II] avec le dérivé réactif d'acide carboxylique de formule [XIII]

$$HOOC—W^1—(X)_e—W^2—(Y)_f—(H) \qquad [XIII]$$

puis avec un composé de formule [XIV]

$$L—W^3—(Z)_g—W^4—CO—R^C \qquad [XIV]$$

selon le même procédé qu'en (ii) ci-dessus pour fournir un composé de formule [I], ou

(vii) la réaction d'un composé de formule [II] avec le dérivé réactif d'acide carboxylique de formule [XV]

$$HOOC—W^1—(X)_e—W^2—(Y)_f—W^3—(Z)_g(H) \qquad [XV]$$

puis avec un composé de formule [XVI]

$$L—W^4—CO—R^C \qquad [XVI]$$

selon le même procédé qu'en (ii) ci-dessus pour fournir un composé de formule [I]; et de plus la conversion de $R^B$, $R^C$, X, Y et Z en d'autres groupes fonctionnels selon des procédés généraux, au besoin, pour obtenir un composé désiré de formule [I].

2. Un procédé selon la revendication 1, caractérisé en ce que l'on prépare un composé répondant à la formule suivante

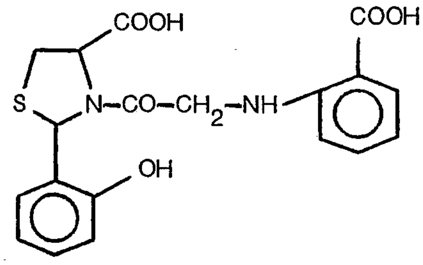

3. Un procédé selon la revendication 1, caractérisé en ce que l'on prépare un composé de formule [I] dans laquelle $R^4$ est un hydrogène.

4. Un procédé selon la revendication 1, caractérisé en ce que l'on prépare un composé de formule [I] dans laquelle $R^b$ est un 2-phényléthyle, un phényle, un 2-fluorophényle, un 3-fluorophényle, un 4-fluorophényle, un 2-nitrophényle, un 3-nitrophényle, un 4-nitrophényle, un 2-chloro-5-nitrophényle, un 2-hydroxyphényle, un 3-cyanophényle, un 4-cyanophényle ou un 2-hydroxy-5-sulfamoylphényle.

5. Un procédé selon la revendication 1, caractérisé en ce que l'on prépare un composé de formule [I] dans laquelle $R^c$ est un hydroxy, un méthoxy, un éthoxy, un hydroxyamino ou

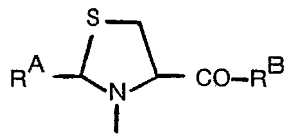 ;

6. Un procédé selon la revendication 1, caractérisé en ce que l'on prépare un composé de formule [I] dans laquelle $R^d$ est un hydrogène, un méthyle, un 2-phényléthyle ou un phényle.

7. Un procédé selon la revendication 1, caractérisé en ce que l'on prépare un composé de formule [I] dans laquelle W est $—CH(CH_3)—CH_2—$, $—CH(CH_3)—(CH_2)_2—$, $—(CH_2)_{1-8}$, $—(CH_2)_{10}—$ ou $—(CH_2)_{12}—$.

8. Un procédé selon la revendication 1, caractérisé en ce que l'on prépare un composé de formule [I] dans laquelle W est $—CH_2COCH(COCH_3)—$.

9. Un procédé selon la revendication 1, caractérisé en ce que l'on prépare un composé de formule [I] dans laquelle W est

$—CH_2—O—CH_2—$, $—(CH_2)_2—O—(CH_2)_2—$, $—(CH_2)_2—S—(CH_2)_2—$
ou $—(CH_2)_2—S—(CH_2)_2—S—(CH_2)_2—$.

10. Un procédé selon la revendication 1, caractérisé en ce qu'on prépare un composé de formule [I] dans laquelle W est

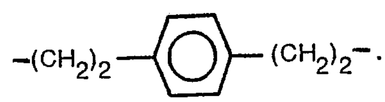

11. Un procédé selon la revendication 1, caractérisé en ce que l'on prépare un composé de formule [I] dans laquelle W est

$—CH(CH_3)—S—CH(Ph)—$, $—CH_2—S—CH(CH_2CH_2Ph)—$, $—CH(CH_3)—S—CH(CH_2CH_2Ph)—$,
$—CH(CH_2Ph)—S—CH_2—$ ou $—CH(CH_2CH_2Ph)—S—CH_2—$.

12. Un procédé selon la revendication 1, caractérisé en ce que l'on prépare un composé de formule [I] dans laquelle W est

$-CH_2NHCH_2-$, 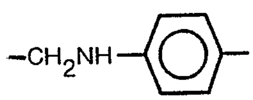 ou $-CH_2NH-CH(CH_2CH_2Ph)-$.

13. Un procédé selon la revendication 1, caractérisé en ce que l'on prépare l'acide (4R)-3-[8-(éthoxycarbonyl)-octanoyl]-2-(3-nitrophényl)-4-thiazolidinecarboxylique.

14. Un procédé selon la revendication 1, caractérisé en ce que l'on prépare le (4R,4'R)-3,3'-(nonanedioyl)bis[2-(3-nitrophényl)-4-thiazolidinecarboxylate de méthyle].

15. Un procédé selon la revendication 1, caractérisé en ce qu'on prépare l'acide (4R)-3-(11-carboxyundécanoyl)-2-(3-cyanophényl)-4-thiazolidinecarboxylique.

16. Un procédé selon la revendication 1, caractérisé en ce que l'on prépare l'acide (4R,4'R)-3,3'-(décanedioyl)bis[2-(3-cyanophényl)-4-thiazolidinecarboxylique].

17. Un procédé selon la revendication 1, caractérisé en ce que l'on prépare l'acide (4R,4'R)-3,3'-dodécanedioyl)bis[2-(3-cyanophényl)-4-thiazolidinecarboxylique].

18. Un procédé selon la revendication 1, caractérisé en ce que l'on prépare le (4R)-3-(8-carboxyoctanoyl)-2-(3-nitrophényl)-4-thiazolidinecarboxylique.

19. Un procédé selon la revendication 1, caractérisé en ce que l'on prépare l'acide (4R,4'R)-3,3'-(nonanedioyl)bis[2-(3-nitrophényl)-4-thiazolidinecarboxylique].

20. Un procédé selon la revendication 1, caractérisé en ce que l'on prépare l'acide (4R)-3-(7-carboxyheptanoyl)-2-(2-hydroxyphényl)-4-thiazolidinecarboxylique.

21. Un procédé selon la revendication 1, caractérisé en ce que l'on prépare l'acide [[(1-carboxy-3-phénylpropyl)amino]acétyl]-2-(2-hydroxyphényl)-4-thiazolidinecarboxylique.

22. Un procédé selon la revendication 1, caractérisé en ce que l'on prépare l'acide (4R)-[[(1-carboxy-3-phénylpropyl)thio]acétyl]-2-(2-hydroxyphényl)-4-thiazolidinecarboxylique.

23. Un procédé selon la revendication 1, caractérisé en ce que l'on prépare l'acide (4R)-3-(4-carboxybutanoyl)-2-(2-hydroxyphényl)-4-thiazolidinecarboxylique.